# EUROPEAN PATENT SPECIFICATION

(11) **EP 3 060 195 B1**
(45) Date of publication and mention of the grant of the patent: **26.09.2018**
(21) Application number: 14816108.6
(22) Date of filing: 24.10.2014
(51) Int. Cl.: A61K 8/64, A61K 8/73, A61Q 19/00, A61K 8/81, A61K 8/86, A61Q 19/08, A61K 8/97, A61K 8/02

(54) **COSMETIC COMPOSITION BASED ON HYALURONIC ACID, METHOD OF PREPARATION AND USE THEREOF**
KOSMETISCHE ZUSAMMENSETZUNG AUF DER BASIS VON HYALURONSÄURE, VERFAHREN ZUR HERSTELLUNG UND VERWENDUNG
COMPOSITION COSMÉTIQUE À BASE D'ACIDE HYALURONIQUE, PROCÉDÉ DE PRÉPARATION ET D'UTILISATION DE CELLE-CI

(30) Priority: 25.10.2013 CZ 20130820
(43) Date of publication of application: 31.08.2016
(73) Proprietor: Contipro a.s., 56102 Dolní Dobrouc (CZ)
(72) Inventor: KNOTKOVA, Katerina, CZ-56501 Chocen (CZ); SOUKUPOVA, Eva, CZ-68605 Uherske Hradiste (CZ); RUZICKOVA, Jana, CZ-14800 Praha 4 Kunratice (CZ); VELEBNY, Vladimir, CZ-56401 Zamberk (CZ)
(74) Representative: Dvorakova, Martina
(86) International application number: PCT/CZ2014/000120
(87) International publication number: WO 2015/058734

(56) References cited:
- WO-A1-2014/065579
- JP-A- 2004 051 521
- US-A1- 2010 216 211
- US-A1- 2010 254 961
- Veronika Hanosova, Marek Pokorny and Vladimir Velebny: "Electrospinning of Hyaluronic Acid/Polyethylene Oxide Blended Fibers", 2nd International Conference, October 12-14, 2010, Olomouc, Czech Republic , 14 October 2010 (2010-10-14), XP002736391, Retrieved from the Internet: URL:http://www.nanocon.eu/files/proceeding s/nanocon_10/lists/paper.htm [retrieved on 2015-02-23]

## Description

### Field of the Art

The invention relates to a cosmetic composition based on hyaluronic acid which comprises nanofibres containing hyaluronic acid or a pharmaceutically acceptable salt thereof and at least one carrier polymer, preferably polyethylene oxide, polyvinyl alcohol. Further, it relates to the method of production of the composition which is carried out by means of electrostatic spinning of the mixture in water.

### State of the Art

Nanofibres are nano materials having a diameter which is usually smaller than 100 nm, Nano glossary. Nanotechnology Task Force, Berlin: Federal Institute for Materials Research and Testing. 24 March 2011. "Taken from ISO/TS 27687:2008 Nanotechnologies - Terminology and definitions for nano-objects - Nanoparticle, nanofibre and nanoplate.", in most cases, however, the size thereof is within the rasnge of 50 to 800 nm. They may be prepared by various methods. Nowadays, perhaps the most frequently used method is the electrostatic spinning method. It is a very simple and effective method for the preparation of ultrathin polymer fibres the diameter of which may vary from approximately 5 to 500 nm, see J.M.Deitzel et al. Polymer 42 (2001) 261-272. Thanks to the structure thereof, said nanofibrous materials have unique properties which allow their broad use in many fields. They may find use for example as filters, composite bracings - see e.g. M.M.Bergshoef et al. Adv. Mater. 11 (1999) 1362-1365, drug carriers - see e.g. E. R. Kenawy et al. J.Control. Release 81 (2002) 57-64 or scaffolds - see e.g. P.Wutticharoenmongkol et al. J. Nanosci. Nanotech. 6(2006)514-522. Nanofibres are prepared from natural, as well as from synthetic polymers. Frequently used natural polymers include polysaccharides, such as hyaluronic acid, cellulose or chitosan, the synthetic ones include polyvinyl alcohol (PVA), polycaprolacton (PCL), polymeric lactic acid (PLA), polyethylene oxide (PEO), acetate cellulose (CA), nylon (NY), or polyurethanes (PU).

Presently, the use of nanomaterials in the form of carriers is common in cosmetics, there are many products on market already, mostly facial creams or serums, containing these materials. More specifically, these are e.g. Revitalift (L'Oreal) which contains Pro-retinol A in the form of nanosoms, then Advanced Night Repair Protective (Estée Lauder) which contains liposoms, Platinéum (Lancôme) containing Hydroxyapatit in the form of nanoparticles and the like. However, the use of nanofibres in cosmetics is a new trend which is still developing. Up to now, there is no cosmetic product known to be present on the market which would be formed by nanofibres. Currently, one of the potential possibilities of using the nanofibres in cosmetics is spinning the active substances together with selected and compatible polymers. Nanofibres may thus be of use as carriers which help in improving the skin condition and alleviating e.g. indications of ageing thereof. Even though there has been no cosmetic product based on nanifibres on the market so far, several possibilities of composition and preparation of such products have been described in literature. One of the first ones who dealt with the preparation of nanofibres for cosmetic applications was Taepaiboon et al. They linked vitamins A and E to fibres of acetate cellulose prepared by the method of electrostatic spinning. Then they monitored by an in vitro method the characteristics and difference in releasing said substances from acetate cellulose nanofibrous supports, as well as from films prepared by pouring off the acetate cellulose solution - see European Journal of Pharmaceutics and Biopharmaceutics, 67 (2007) 387-397. In 2010 Fathi-Azarbayjani et al. published the preparation of nanofibrous anti-wrinkle facial masks of PVA and cyclodextrins (RM beta-CDs) by the method of electrostatic spinning, which contain vitamins A and C (retinoic and ascorbic acid), nanoparticles of gold and collagen. It is a mask which is in a dry state and therefore the substances linked therein do not undergo hydrolysis. Upon placing the mask on face and moistening thereof, the mask is dissolved within 15 minutes, it releases the active substances and ensures the maximum penetration thereof - see A. Fathi-Azarbayjani et al. AAPS PharmSciTech, 11 (2010) 1164-1170. In this case, the mask is not deposited on any support backup. The use of nanofibres as carriers of active substances in connection with the possible application in the field of cosmetics was also published in 2013 by Madhaiyan et al. and Sheng et al. In the first case, the nanofibres were prepared from a biocompatible polymer polycaprolactone (PCL), to which vitamin B₁₂ was bound - see K.Madhaiyan et al International Journal of Pharmaceutics, 444 (2013) 70-76. In the second case, the fibres were prepared from fibroin obtained from silk, to which vitamin E was bound - see X.Sheng et al. International Journal of Biological Macromolecules (2010), doi:10.1016/j.ijbiomac.2013.01.029. Up till now, no publication has been published which would describe spinning of cosmetically active substances other than vitamins, or inorganic particles or collagen. Polymer substances (acetate cellulose, polyvinyl alcohol, cyclodextrins, polycarpolacton and fibroin), which were used in the above mentioned publications for the preparation of nanofibres are allowed in cosmetics, but, however, they do not belong to the frequently used basic materials. These substances do not have any improving effect on the skin, the presence thereof in the preparations is only additive, they serve first of all for forming the film or for adjusting the viscosity.

Korean patent application KR2011110482A describes nanofibres made of hyaluronic acid, containing encapsulated vitamin as a prevention of degradation of the vitamin caused by the sunlight. More specifically, it discloses fibres made of hyaluronic acid containing a vitamin and a water-soluble polymer. The vitamin may be e.g. A or E, the water-soluble polymer is hyaluronic acid. The method of preparation of nanofibres consists in the preparation of a mixture of the vitamin solution and the water-soluble polymer and in electrostatic spinning. The solution of the vitamin is prepared by dissolving the vitamin in ethanol in the presence of a surfactant (such as ceteth and nikkol), the presence of which in the preparation for cosmetic use is undesirable. The international patent application WO2009045042A discloses the preparation of nanofibres from mixtures of various polymers, including e.g. polyurethane, polyacrylonitril, nylon, polymeric lactic acid, polycarbonate, polycaprolacton and others. Hyaluronic acid was not used in this case. To these fibres, extracts and/or natural essential oils were bound. The possibility of use of said fibres in cosmetics is mentioned.

Hyaluronic acid, unlike all the above mentioned synthetic polymers, is a substance which is body-innate, it is a part of connective tissues and skin where the main property thereof is to bond water and by this to maintain sufficient hydration. However, with ageing the content thereof in the skin decreases, the hydration is reduced and thereby wrinkles and other signs of ageing occur. That is why hyaluronic acid is added to various cosmetic preparations which are designated for topic, as well as intradermal applications.

As far as dry cosmetics are concerned, the Japanese patent application No. JP2006182750A discloses the preparation of cosmetics by means of freeze-drying (lyophilisation). It means drying of an aqueous solution containing a mixture of collagen, hyaluronic acid and an ascorbic acid derivative. This mixture is then re-dissolved in water or skin lotion prior to use. Said form of cosmetics is characterized by a long stability and absence of preservatives. Therefore, it is a type of dry cosmetics, however, it is such that it is not formed by nanofibres. The drawback thereof, as compared to the presented nanofibrous cosmetics, is that it's time- and manipulation-demanding and the preparation thereof associated with the lyophilisation is expensive. The step of re-dissolving the lyophilisate extends the time which the customer must spend by the home preparation of the product prior to its application itself.

Another Japanese patent application No. JP2004051521A deals with cosmetics comprising a dry cosmetic crude material on a non-woven fibre. The dry crude material may be collagen, vitamin A and C, or hyaluronic acid. Further, the product contains a thickening agent which may be water-soluble alginate, a cellulose derivative, chitosan, chitin, PVA and PVP. The fibre is made of non-woven silk which is filled with natural silk.
US patent application No.2010/254961 mentions very low content of HA 0,01 wt.% in nanofibers used for cosmetic purposes wherein HA acts as a humectant.

Furthermore it is published the abstract of presentation in form of a poster Hanosova et al. "Electrospinning of Hyaluronic Acid/Polyethylene Oxide Blended Fibers" in 2nd International Conference October 14, 2010. I describes only generally a possibility of production of PEO/HA blend nanofibrous membranes. There is no mention about possibility to produce cosmetic composition wherein the content of HA in fibers is high without presentation of undesirable beads on fibers.

US patent application No.2010/216211 relates to fibrous mats comprising chitosan nanofibers substantionally free of fibers of PEO or PVA. Moreover chitosan is substantionally insoluble in water.

### Summary of the Invention

The drawbacks resulting from the state-of-the-art solutions are overcome by the cosmetic composition as defined in Claim 1. The cosmetic composition based on hyaluronic acid includes nanofibres comprising at least 1 wt.% of hyaluronic acid or a pharmaceutically acceptable salt thereof and at least one carrier polymer.

The advantage of the cosmetic composition is a high content of hyaluronic acid in nanofibres, i.e. up to 90 wt.% or even up to 99 wt.%. As mentioned above, hyaluronic acid or a salt thereof is more natural for the skin than other commonly used polymers for the preparation of nanofibres and, as opposed to them, it has a significant positive effect on the skin. The increased concentration thereof smoothes out wrinkles. Moreover, in commercially available creams the HA concentration is up to 24 times lower when applied, and in commercially available facial serums in liquid form the HA concentration is up to 13 times lower when applied, than when using the dry composition according to the invention.

Further, as opposed to the state-of-the-art nanofibrous products it can contain any cosmetic active component, not only vitamins or inorganic particles.

Moreover, it does not contain any preservatives, stabilizing agents or residues of solvents, it has a long-term stability, its solubility in water is very good and it does not require any further modification prior to its application.

The pharmaceutically acceptable salt is selected from the group including any of alkali metal ions, preferably Na⁺, K⁺. The carrier polymer is selected from the group containing polyethylene oxide, polyvinyl alcohol, preferably polyethylene oxide. The carrier polymers according to the invention enable to carry out the spinning just in water, without the presence of other polar solvents the presence of which in nanofibres could irritate or dehydrate the skin upon the application of the agent.

The nanofibres comprised in the composition according to the invention have a diameter within the range of 1 to 400 nm, preferably 20 to 300 nm, more preferably 50 to 200 nm.

According to a preferred embodiment of the invention, the nanofibres included in the composition further comprise a cosmetically active substance. That can be any water-soluble or insoluble substances of various chemical composition and origin which are designated for use in cosmetics. Preferably, the cosmetically active substance according to the invention is selected from the group including
- water-soluble peptides or water-soluble proteins, more preferably selected from the group comprising acetyl hexapeptide-8, sh-hexapeptide-1, whey protein, soya protein, palmitoyl tripetide-5, palmitoyl hexapeptide or tripeptide-32,
- water-soluble polysaccharides, more preferably selected from the group including more preferably selected from the group containing schizophyllan, sodium carboxymethyl beta-glucane, sodium caproyl hyaluronate or glucomannan,
- water-soluble plant extracts, more preferably selected from the group containing an extract from above-ground parts of Epilobium angustifolium in 1,3-butanediol, an extract from green seeds of Coffea arabica in 1,3-butanediol, an extract from the leave of Camellia Sinensis in 1,2-propanediol, an extract from the root of Panax Ginseng in 1,2-propanediol, an extract from Aloe Barbadensis in water, plankton extract in water,
- water-soluble polyols, more preferably selected from the group containing propylene glycol, butylene glycol, glycerol;
- water-soluble vitamins, more preferably selected from the group containing D-panthenol, ethyl ester of ascorbic acid,
- Micrococcus lysate, which is a lysate of bacteria Micrococcus luteus, further creatine, 1-methylhydantoin-2-imide, sodium lactate, glycin sodium salt of pyroglutamic acid, fructose, urea, niacine amide, inositol, sodium benzoate, lactic acid or other substancess.

Further, the nanofibres of the composition according to the invention may contain an adjuvant, preferably carboxymethyl cellulose. Another component of the nanofibrous composition according to the invention may be a lipophilic substance in the form of micelles. The water-insoluble substances are spun either in the form of a suspension or polymeric micelles, where the hydrophobic substance is encapsulated in a water-soluble polymer. The lipophilic substance is preferably selected from the group comprising a resin extract from the tree Commiphora Mukul, an extract from Lavadula Stoechas in triglycerides of octanoic acid and decanoic acid, coenzyme Q10, ascorbic acid palmitate, pyridoxine dipalmitate, tocopheryl acetate, glycyrrhetinoic acid, cholesterol.

Another preferred embodiment of the invention is a composition including nanofibres containing hyaluronic acid or salts thereof and polyethylene oxide, wherein the weight ratio of hyaluronic acid or the salt thereof with respect to polyethylene oxide is 1/99 to 99/1, preferably 1/99 to 90/10, more preferably 5/95 to 85/15, most preferably 50/50 to 80/20.

Another preferred embodiment of the invention is a composition including nanofibres containing hyaluronic acid or salts thereof and polyvinyl alcohol, wherein the weight ratio of hyaluronic acid or the salt thereof with respect to polyvinyl alcohol is 2/98 to 50/50, preferably 5/95 to 50/50, more preferably 20/80 to 50/50.

A composition including nanofibres containing hyaluronic acid or salts thereof and a mixture of polyethylene oxide, polyvinyl alcohol and carboxymethyl cellulose, wherein the content of hyaluronic acid or the salt thereof is from 1 to 80 %wt., preferably 1 to 50 %wt., more preferably 4.4 %wt. The weight ratio of carboxymethyl cellulose, polyethylene oxide and polyvinyl alcohol in the mixture is from 1/1/0.01 to 1/1/1, preferably 1/1/0.5.

The molecular weight of hyaluronic acid or a pharmaceutically acceptable salt thereof contained in the nanofibres included in the composition is within the range of 1x10⁴ to 3 x 10⁵ g/mol, preferably 5x10⁴ to 1.5 x 10⁵ g/mol, wherein their content in the dry matter of the nanofibres is preferably at least 1 wt.%, more preferably at least 4.4 wt.% and most preferably at least 50 %.

The content of hyaluronic acid or a pharmaceutically acceptable salt thereof is within the range of 2 to 90 %wt., preferably 4.4 to 80 %wt., more preferably 50 to 80 %wt.

The molecular weight of the carrier polymer contained in the nanofibres included in the composition according to the invention is within the range from 1 x10⁴ to 9x10⁵ g/mol, wherein the molecular weight is preferably for polyethylene oxide within the range of 3 x 10⁵ to 9 x 10⁵ g/mol or for polyvinyl alcohol within the range of 1 x 10⁴ to 4 x 10⁵ g/mol.

The content of the carrier polymer in the dry matter of the nanofibres included in the composition which contains nanofibres comprising hyaluronic acid or a pharmaceuticallly acceptable salt thereof and a mixture of polyethylene oxide, polyvinyl alcohol and carboxymethyl cellulose according to the invention is within the range of 0.001 to 90 %wt., preferably 5 to 65 %wt., wherein the content is preferably for polyethylene oxide within the range of 5 to 90 %wt., preferably 10 to 50 %wt. or for polyvinyl alcohol within the range of 0.001 to 30 %wt., preferably 5 to 25 %wt..

Further, it is preferred when the molecular weight of carboxymethyl cellulose contained in the nanofibres included in the composition according to the invention is within the range of 1 x 10⁵ to 4 x 10⁵ g/mol, preferably 1.5 x 10⁵ to 3 x 10⁵ g/mol, wherein the content thereof is within the range of 0.001 to 50 %wt., preferably 10 to 40 %wt.

Further, it is preferred when the content of the cosmetically active substances in the dry matter of the nanofibres included in the composition according to the invention is within the range of 0.001 to 50 %wt., wherein preferably
for the water-soluble peptides it is within the range of 0.001 to 10 %wt. or
for the water-soluble polysaccharides it is within the range of 0.005 to 0,5 %wt. or
for the water-soluble plant extracts it is within the range of 0.01 to 10 %wt. or
for the water-soluble polyols it is within the range of 0.1 to 45 %wt. or
for the water-soluble vitamins it is within the range of 0.1 to 5 %wt.

The composition according to the invention is in the form of a layer, either as a self supporting layer or it is deposited on a support. In case of using a support, the composition according to the invention is used as a facial mask. In case of not using any support the composition according to the invention is used as a facial serum or a facial cream and it may also be used as a self supporting facial mask. The composition according to the invention is in a water-soluble dry form.

The areal weight of a layer of the composition according to the invention for use in cosmetics on a support is within the range of 0.2 to 50 g/m², preferably 1.5 to 20 g/m², more preferably 1.0 to 20 g/m², most preferably 1.3 to 1.7 g/m² or 1.7 g/m². The areal weight of a layer of the self-supporting composition according to the invention is within the range of 2 to 50 g/m², preferably 4 to 20 g/m². A layer of the composition according to the invention having a lower areal weight may be used for an every-day care, where the concentration of the substances is only replenished. A layer of the composition according to the invention having a higher areal weight will then be needed, from the cosmetic point of view, for a more intense course. Areal weights of layers of compositions according to this application were determined by means of small samples - standard: ČSN EN 12127 (80 0849).

In case the composition according to the invention is deposited on a support, then the support is meant to be a textile which is preferably selected from the group comprising a woven fabric, warp-knitted fabric, load knitted fabric, non-woven textile or a foil.

The material of the textile in the form of a support according to the invention is selected from the group comprising polyester, cellulose, polyurethane, polypropylene, polyethylene, viscose, polyamide or mixtures thereof.

The material of the foil in the form of the support according to the invention is selected from the group comprising aluminium, such as an aluminium foil, polyamide, polyester, polypropylene, polyethylene or mixtures thereof.

Another embodiment according to the invention is a method of the production of the composition as described above, wherein a spinning solution is prepared, comprising hyaluronic acid, a salt thereof and at least one carrier polymer in water, thereafter it is spun electrostatically. A part of the spinning solution may be an optional addition of a cosmetically active substance and/or an adjuvant, as described above.

Another preferred embodiment of the method according to the invention is that micelles containing a lipophilic substance or a mixture thereof, as described above, are added to the spinning solution. The spinning may as well be carried out from a suspension which is formed after the addition of the lipophilic substance or the mixture thereof into the spinning solution.

The components of the spinning solution are homogenized in water for 10 to 16 hours, preferably for up to 12 hours.

Yet another preferred embodiment of the method according to the invention is that a spinning solution is prepared, comprising hyaluronic acid or a salt thereof and polyethylene oxide or comprising hyaluronic acid and a mixture of polyethylene oxide, carboxymethyl cellulose and polyvinyl alcohol, wherein it forms 2 to 12 %wt., preferably 4 to 10 %wt., of the spinning solution.

The method of preparation of the composition according to the invention allows using hyaluronic acid in a broad scale of molecular weights and in a high concentration and at the same time it allows for a great variability of the concentrations of the individual cosmetic crude materials. The method of production according to the invention proceeds at a temperature falling within the range of 18 to 45 °C and normal pressure from an aqueous medium in electrostatic field, where a bundle of fibres collected on a collector covered with a support, i.e. a textile or a foil, is drawn out of the spinning solution by means of an electrostatic force. Such an arrangement facilitates the collection of the layer of fibres and at the same time of the textile or foil, then the obtained composition is in a supporting form. The self-supporting composition is obtained by the same above described method according to the invention without using the support.

Thanks to that the whole process is carried out from water as a solvent, no enhanced demands on the device, the preparation of the solutions or operational safety are associated therewith.

The compositions prepared in this way are suitable for topical use in cosmetics. The fibres deposited on the carrier support represent dry facial masks, the self-supporting composition according to the invention then represents a dry form of a facial serum, but at the same time it may be used, like the composition deposited on a support, as dry facial masks.

The advantage of the composition according to the invention is its dry form, which allows omitting any preservatives, and at the same time the stability of the product is ensured. Other advantages also include the absence of emulsifiers. After the application, no greasy film is left on the skin. The advantage is also the immediate solubility of the composition when in contact with water. It is not necessary to dissolve or to treat in any other way the product prior to its application on the skin.

The method of application of the dry composition according to the invention on a support as a facial mask includes several steps: moistening the face, placing the product on the moistened skin for 5-30 minutes, wherein 10 minutes are preferred, then removing the support material from the skin and washing the dry film of the mask by means of water. It is recommended to apply a regular cream after the application.

The method of application of the dry composition according to the invention without the support as a facial mask includes the following steps: moistening the face by water by means of a diffuser, optionally by means of sponges; taking the product out of the wrapping by means of tweezers or fingers; applying the product on the skin and spreading the product all over the face and neck. The mask is let to interact for 5 - 20 minutes, then the dry film of the mask is washed by means of water. It is recommended to apply a regular cream after the application.

The method of application of the dry composition according to the invention without the support as a facial serum includes these steps: moistening the face by water, optimally by means of a diffuser, taking the product out of the wrapping by means of tweezers, applying the product on the skin and spreading the product all over the face, neck and optionally the neck line. The product remains on the skin, it is not washed off. However, it is necessary to use a sufficient amount of water and spred it thoroughly, so that no visible film remains on the skin.

### Definitions of the Terms

The term "layer" means a layer of the composition according to the invention which is formed on the collecting electrode after the spinning of the polymeric mixture.

The term "foil" usually means a very thin sheet, produced industrially from synthetic polymers and metals.

The term "sh-hexapeptide-1" means a peptide the sequence of which contains 6 amino acids. Said sequence is as follows - H-GSPAGS-OH (glycine - serine - proline - alanine - glycine - serine).

The term "carrier polymer" means a biocompatible naturalor synthetic polymer which may be spun by itself electrostatically, is suitable for cosmetic applications and does not cause any undesirable or systemic toxicity.

The term "water-soluble peptides" means oligopeptides containing up to 10 amino acids or polypeptides with up to 100 amino acids which are soluble in water.

The term "water-soluble proteins" means high-molecular biopolymers containing more than 100 amino acids or having the molecular weight within the range of 200 to 180 000 g/mol, which are soluble in water.

The term "water-soluble polysaccharides" means polymeric saccharides having the molecular weight within the range of 200 000 - 1 500 000 g/mol, which are soluble in water.

The term "water-soluble plant extracts" means alcoholic extracts from plants or their parts which are suitable for cosmetic applications, do not cause any undesirable or systemic toxicity and which are soluble in water.

The term "water-soluble polyols" means multiple-hydric alcohols, preferably diols, which are soluble in water.

The term "water-soluble vitamins" means vitamins of the group B, i.e. B1, B2, B3, B5, B6, H (B7), B9, B12 or vitamin C, or esteric derivatives thereof.

### Brief Description of the Drawings

**Fig. 1****:** A nanofibrous layer comprising hyaluronic acid, polyethylen oxide and whey protein.
**Fig. 2****:** A nanofibrous layer comprising hyaluronic acid, polyethylene oxide, acetyl hexapeptide-8 and whey protein.
**Fig. 3****:** A nanofibrous layer comprising hyaluronic acid, polyethylene oxide, acetyl hexapeptide-8 and whey protein, an extract from the aerial parts Epilobium angustifolium, an extract from green seeds of Coffea arabica, an extract from the leaf of Camellia Sinensis, an extract from the root of Panax Ginseng.
**Fig. 4****:** A nanofibrous layer comprising hyaluronic acid, polyethylene oxide, acetyl hexapeptide-8 and whey protein, an extract from the above-ground parts of Epilobium angustifolium, an extract from green seeds of Coffea arabica, an extract from the leaf of Camellia Sinensis, an extract from the root of Panax Ginseng.
**Fig. 5A****:** A nanofibrous layer obtained by spinning the mixtures: sample A in its composition contains HA/PEO and the substances from the group A - see Table 2 below.
**Fig. 5B****:** A nanofibrous layer obtained by spinning the mixtures: sample B in its composition contains HA/PEO and the substances from the groups A + B - see Table 2 below.
**Fig. 5C****:** A nanofibrous layer obtained by spinning the mixtures: sample C in its composition contains HA/PEO/CMC/PVA and the substances from the group A - see Table 2 below.
**Fig. 5D****:** Sample D in its composition contains HA/PEO/CMC/PVA and the substances from the groups A + B - see Table 2 below.
**Fig. 6A****:** A nanofibrous layer obtained by spinning the mixtures: sample A in its composition contains HA/PEO and the substances from the group A - see Table 3 below
**Fig. 6B****:** A nanofibrous layer obtained by spinning the mixtures: sample B in its composition contains HA/PEO and the substances from the groups A + B - see Table 3 below.
**Fig. 6C****:** A nanofibrous layer obtained by spinning the mixtures: sample C in its composition contains HA/PEO/CMC/PVA and the substances from the group A - see Table 3 below.
**Fig. 6D****:** Sample D in its composition contains HA/PEO/CMC/PVA and the substances from the groups A + B - see Table 3 below.

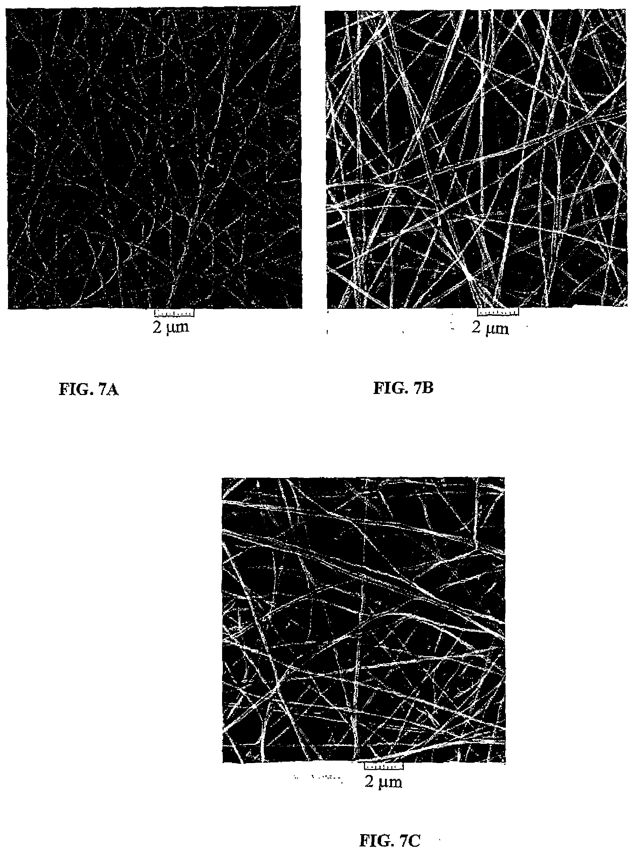

**Fig. 7****:** A nanofibrous layer on various support materials A) low-density PE (rough) B) woven fabric - cellulose C) knitted fabric - cellulose.
**Fig. 8****:** H¹ NMR spectrum of creatine in the mixture of HA/PEO
**Fig. 9****:** A nanofibrous layer comprising hyaluronic acid, polyethylene oxide and creatine
**Fig. 10****:** H¹ NMR spectrum of creatine in the mixture of HA/PEO
**Fig. 11****:** A nanofibrous layer comprising hyaluronic acid, polyethylene oxide and creatine
**Fig. 12****:** A nanofibrous layer comprising hyaluronan with polymer micelles containing pyridoxine dipalmitate and polyethylene oxide.
**Fig. 13****:** The ratio of soya proteins and water in mg to the weight of the sample in mg in the nanofibrous layer.
**Fig. 14****:** A nanofibrous layer obtained by spinning the mixture with soya proteins.
**Fig. 15****:** The ratio of acetyl hexapeptide-8 in mg to the weight of the sample in the nanofibrous layer.
**Fig. 16****:** A nanofibrous layer obtained by spinning the mixture with acetyl hexapeptide-8 and water.
**Fig. 17****:** A nanofibrous layer obtained by spinning the mixture of hyaluronic acid, polyethylene oxide, whey protein, propylen glycol, an extract of aerial parts of Epilobium angustifolium, aloe barbadensis and sodium carboxymethyl beta-glucan.
**Fig. 18****:** Effect of the products of instant cosmetics in the form of a serum and cream serum on the elasticity of the skin during 3 hours after the application.
**Fig. 19****:** Effect of the products of instant cosmetics in the form of a serum and cream serum on the elasticity of the skin during 24 hours after the application.
**Fig. 20****:** Effect of the products of instant cosmetics in the form of a serum and cream serum on the elasticity of the skin during a 4-week application.
**Fig. 21****:** Effect of the products of instant cosmetics in the form of a serum and cream serum on the values of the serum on the skin during 2 hours after the application.
**Fig. 22****:** Effect of the products of instant cosmetics in the form of a serum and cream serum on the values of TEWL during a 4-week application.
**Fig. 23****:** Effect of the products of instant cosmetics in the form of a facial mask on the elasticity of the skin during 3 hours after the application.
**Fig. 24****:** Effect of the products of instant cosmetics in the form of a facial mask on the values of TEWL during 3 hours after the application.

### Preferred Embodiments of the Invention

The nanofibrous instant cosmetics was prepared on an apparatus 4Spin 4SPIN® C4S LAB, generally with the distance of electrodes of 20 cm and dosage 150 µl/min. The voltage was within the range of 20 - 60 kV.

The images from the scanning electron microscope were made on the apparatus Tescan VEGA II LSU with a tungsten cathode and the maximum resolution of 3 nm. The images were made in the high vacuum regime. The accelerating voltage was 5 kV.

NMR spectra of the additives were measured on BRUKER AVANCE 500 (500 MHz) in deuterated water. For processing the experimental data, software of the company Bruker TOPSPIN 1.2 and software 25 SpinWorks 3.1 were used.

The qualitative and quantitative determination of the extracts was performed by means of HPLC Shimadzu Prominence LC-20 equipped with a UV-Vis detector.

The qualitative and quantitative determination of the peptides as active substances was performed by means of LC Waters Acquity equipped with a mass spectrometer MALDI-SYNAPT Q-TOF.

The UV-Vis spectra were measured on a UV-Vis spectrophotometer Varian Cary 100 Cone within the wavelength range of 800 - 190 nm, optionally 400 - 190 nm.

**Table 1: Examples of active substances and recommended amount thereof**

| **Active substance** | **Recommended range of concentrations in the composition** |
|---|---|
| whey protein | 0.5 % |
| acetyl hexapeptide-8, | 3 - 10 % |
| (1%)extract from the above-ground parts (blossoms, leaves, stem) of the plant Epilobium Angustifolium, butyleneglycol | 0.1 - 2 % |
| 1% Extract from green seeds of Coffea Arabica , butyleneglycol | 0.1 - 2 % |
| - propyleneglycol, extract from the leaves of Cammelia Sinensis | 1 - 10 % |
| propyleneglycol, extract from the root of Panax Ginseng | 1 - 10 % |
| butyleneglycol | 1-10 % |
| glycerol | 2 - 5 % |
| propanediol | 1 - 8 % |
| schizophyllan | 0.005 - 0.2 % |
| sodium carboxymethyl beta-glucane | 0.01 - 0.3 % |
| glucomannan | 0.005 - 0.25 % |
| sodium caproylhyaluronate | 0.02 - 0.25 % |
| Aloe Barbadensis | 1 - 10 % |
| alantoin | 0.2 - 2 % |
| D-panthenol, water | 0.5 - 3 % |
| sodium lactate, a sodium salt of pyroglutamic acid, glycine, fructose, urea, niacinamide, inositol, sodium benzoate, lactic acid | 1 - 2 % |
| creatine | 0.5 - 1.4 % |
| 1-methylhydantoin-2-imide | 0.1 - 1.5 % |
| glyceryl polymethakrylate and propyleneglycol and palmitoyl oligopeptide | 2 - 5 % |
| ethyl ester of ascorbic acid | 0.1 - 3 % |
| sh-hexapeptide-1 | 0.001 - 0.01 % |
| soya proteins, water | 3 - 10 % |
| Micrococcus lysate, lecithin, water | 1 - 5 % |
| plankton extract, water, lecithin | 1 - 5 % |
| urea | 0.5 - 10 % |
| resin extract from the tree Commiphora Mukul | 0.1 - 2% |
| triglycerides of octanoic acid and decanoic acid and extract from Lavandula stoechas | 0.1-3% |
| coenzyme Q10 | 2 - 6 % |
| ascorbic acid palmitate | 0.05 - 1% |
| Pyridoxine dipalmitate | 0.5 - 2% |
| Retinyl palmitate | 0.1-1% |

### Example 1

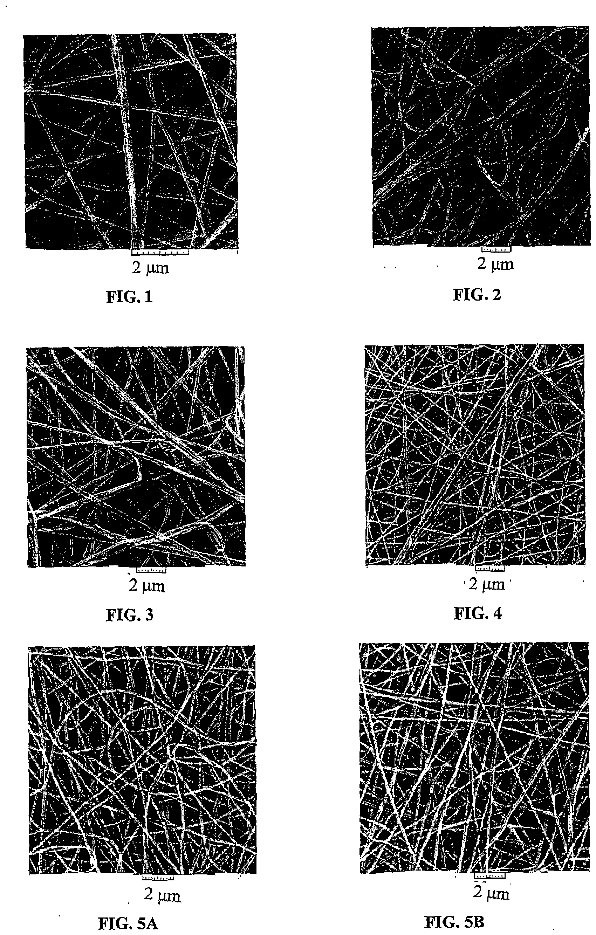

A solution of 3.816 g of hyaluronic acid (HA) having the molecular weight of 83 200 g/mol and 0.96 g of polyethylene oxide having the molecular weight of 400 000 g/mol, 0.024 g of whey protein and 75.2 g of water was prepared. The solution was homogenized for 14 hours at the temperature of 20 °C. The solution was spun on a collecting electrode without any support textile. Fig. 1 shows a photo from the scanning electrone microscope. The areal weight of the nanofibrous layer is 11.023 g/m². The diameter of the fibres is 105 nm.

### Example 2 (HA/PEO 2/98)

A solution of 0.540 g of hyaluronic acid (HA) having the molecular weight of 83 200 g/mol, 5.88 g of polyethylene oxide having the molecular weight of 400 000 g/mol and 94 g of water was prepared. Hyaluronic acid formed 2 % of the dry matter. The solution was homogenized for 10 hours at the temperature of 45 °C. The solution was spun on a collecting electrode without any support textile. The areal weight of the nanofibrous layer is 15.59 g/m². The diameter of the fibres is 144 nm.

### Example 3 (HA/PEO 98/2)

A solution of 5.88 g of hyaluronic acid (HA) having the molecular weight of 83 200 g/mol, 0.06 g of polyethylene oxide having the molecular weight of 400 000 g/mol, 0.06 g of polyethylene oxide having the molecular weight of 4 000 000 g/mol and 94 g of water was prepared. Hyaluronic acid formed 98 % of the dry matter. The solution was homogenized for 12 hours at the temperature of 18 °C. The solution was spun on a collecting electrode without any support textile. The areal weight of the nanofibrous layer is 12.3 g/m². The diameter of the fibres is 179 nm.

### Example 4 (HA/PEO 80/20)

A solution of 4.8 g of hyaluronic acid (HA) having the molecular weight of 83 200 g/mol, 1.2 g of polyethylene oxide having the molecular weight of 400 000 g/mol and 94 g of water was prepared. Hyaluronic acid formed 80 % of the dry matter. The solution was homogenized for 16 hours at the temperature of 20 °C. The solution was spun on a collecting electrode without any support textile. The areal weight of the nanofibrous layer is 14.1 g/m². The diameter of the fibres is 116 nm.

### Example 5

A solution of 3.636 g of hyaluronan (HA) having the molecular weight of 300 000 g/mol and 0.96 g of polyethylene oxide having the molecular weight of 100 000 g/mol, 0.024 g of whey protein, 0.18 g of acetyl hexapeptide-8 and 75.2 g of water was prepared. Hyaluronic acid formed 75.75 % of the dry matter. The solution was homogenized for 12 hours at the temperature of 23 °C. The solution was spun on a collecting electrode without any support textile. Fig. 2 shows a photo from the scanning electrone microscope. The areal weight of the nanofibrous layer is 10.08 g/m². The diameter of the fibres is 131 nm.

### Example 6

A solution of 2.746 g of hyaluronan (HA) having the molecular weight of 112 400 g/mol and 0.72 g of polyethylene oxide having the molecular weight of 600 000 g/mol, 0.018 g of whey protein, 0.108 g of acetyl hexapeptide-8, 0.6 g of the extract from the above-ground parts of Epilobium Angustifolium, 0.6 g of the extract from the green seeds of Coffea arabica, 0.006 g of the extract from the green leaves of Camellia sinensis, 0.006 g of the extract from the root of Panax Ginseng and 55.2 g of water was prepared. Hyaluronic acid formed 57.15 % of the dry matter. The solution was homogenized for 14 hours at the temperature of 20 °C. The solution was spun on a collecting electrode without any support textile. Fig. 3 shows a photo from the scanning electrone microscope. The areal weight of the nanofibrous layer is 10.8 g/m². The diameter of the fibres is 148 nm.

### Example 7

A solution of 0.176 g of hyaluronan (HA) having the molecular weight of 97 700 g/mol and 1.507 g of polyethylene oxide having the molecular weight of 900 000 g/mol, 1.4 g of carboxymethyl cellulose having the molecular weight of 250 000 g/mol, 4.775 g of 16 % polyvinyl alcohol having the molecular weight of 28 000 g/mol, 0.02 g of whey protein (CLR-Berlin), 0.12 g of acetyl hexapeptide-8 (Lipotec s.a.), 0.4 g of the extract from the above-ground parts of Epilobium angustifolium, 0.4 g of the extract from the green seeds of Coffea arabica, 0.004 g of the extract from the green leaves of Camellia sinensis, 0.004 g of the extract from the root of Panax Ginseng and 55.2 g of water was prepared. Hyaluronic acid formed 3.6 % of the dry matter. The solution was homogenized for 16 hours at the temperature of 40 °C. The solution was spun on a collecting electrode without any support textile. Fig. 4 shows a photo from the scanning electrone microscope. The areal weight of the nanofibrous layer is 11.58 g/m². The diameter of the fibres is 88 nm.

### Example 8 (HA/PVA 2/98)

A solution of 0.2 g of hyaluronic acid (HA) having the molecular weight of 83 200 g/mol, 9.8 g of polyvinyl alcohol having the molecular weight of 125 000 g/mol and 90 g of water was prepared. Hyaluronic acid formed 2 % of the dry matter. The solution was homogenized for 13 hours at the temperature of 25 °C. The solution was spun on a collecting electrode without any support textile. The areal weight of the nanofibrous layer is 7.77 g/m². The diameter of the fibres is 290 nm.

### Example 9 (HA/PVA 50/50)

A solution of 5 g of hyaluronic acid (HA) having the molecular weight of 83 200 g/mol, 5 g of polyvinyl alcohol having the molecular weight of 125 000 g/mol and 90 g of water was prepared. Hyaluronic acid formed 50 % of the dry matter. The solution was homogenized for 15 hours at the temperature of 45 °C. The solution was spun on a collecting electrode without any support textile. The areal weight of the nanofibrous layer is 1 g/m². The diameter of the fibres is 180 nm.

### Example 10 (HA/PEO/CMC/PVA 4.5/38.2/38.2/19.1)

A solution of 0.540 g of hyaluronic acid (HA) having the molecular weight of 260 000 g/mol and 4.5840 g of polyethylene oxide having the molecular weight of 400 000 g/mol, 4.5840 g of carboxymethyl cellulose having the molecular weight of 395 000 g/mol, 2.2920 g of polyvinyl alcohol having the molecular weight of 125 000 g/mol and 288 g of water was prepared. Hyaluronic acid formed 4.5 % of the dry matter. The solution was homogenized for 12 hours at the temperature of 25 °C. The solution was spun on a collecting electrode without any support textile. The areal weight of the nanofibrous layer is 11.023 g/m². The diameter of the fibres is 105 nm.

### Example 11 (HA/PEO/CMC/PVA 80/10/5/5)

A solution of 1.623 g of hyaluronic acid (HA) having the molecular weight of 83 200 g/mol and 0.213 g of polyethylene oxide having the molecular weight of 400 000 g/mol, 0.103 g of carboxymethyl cellulose having the molecular weight of 250 000 g/mol, 0.998 g of polyvinyl alcohol having the molecular weight of 125 000 g/mol and 48 g of water was prepared. Hyaluronic acid formed 80 % of the dry matter. The solution was homogenized for 10 hours at the temperature of 40 °C. The solution was spun on a collecting electrode with a support textile (spunbund polypropylene 18 g/m²). The areal weight of the nanofibrous layer is 6.23 g/m². The diameter of the fibres is 96 nm.

### Example 12 (HA/PEO/CMC/PVA 1/40/40/19)

A solution of 0.023 g of hyaluronic acid (HA) having the molecular weight of 83 200 g/mol and 0.821 g of polyethylene oxide having the molecular weight of 400 000 g/mol, 0.804 g of carboxymethyl cellulose having the molecular weight of 250 000 g/mol, 0.382 g of polyvinyl alcohol having the molecular weight of 125 000 g/mol and 48 g of water was prepared. Hyaluronic acid formed 1 % of the dry matter. The solution was homogenized for 15 hours at the temperature of 22 °C. The solution was spun on a collecting electrode with a support textile (spunbund polypropylene 18 g/m²). The areal weight of the nanofibrous layer is 13.82 g/m². The diameter of the fibres is 62 nm.

### Example 13 (HA/PEO/CMC/PVA 19.6/40/40/0.4)

A solution of 0.392 g of hyaluronic acid (HA) having the molecular weight of 83 200 g/mol and 0.817 g of polyethylene oxide having the molecular weight of 400 000 g/mol, 0.814 g of carboxymethyl cellulose having the molecular weight of 250 000 g/mol, 0.008 g of polyvinyl alcohol having the molecular weight of 125 000 g/mol and 48 g of water was prepared. Hyaluronic acid formed 19.6 % of the dry matter. The solution was homogenized for 12 hours at the temperature of 22 °C. The solution was spun on a collecting electrode with a support textile (spunbund polypropylene 18 g/m²). The areal weight of the nanofibrous layer is 7.35 g/m². The diameter of the fibres is 57 nm.

### Example 14 (HA/PEO/CMC/PVA 10/30/30/30)

A solution of 0.205 g of hyaluronic acid (HA) having the molecular weight of 83 200 g/mol and 0.613 g of polyethylene oxide having the molecular weight of 400 000 g/mol, 0.609 g of carboxymethyl cellulose having the molecular weight of 250 000 g/mol, 0.598 g of polyvinyl alcohol having the molecular weight of 125 000 g/mol and 48 g of water was prepared. Hyaluronic acid formed 10 % of the dry matter. The solution was homogenized for 14 hours at the temperature of 40 °C. The solution was spun on a collecting electrode with a support textile (spunbund polypropylene 18 g/m²). The areal weight of the nanofibrous layer is 9.09 g/m². The diameter of the fibres is 54 nm.

### Example 15

Solutions of a solution of hyaluronic acid having the molecular weight of 150 000 g/mol and polyethylene oxide having the molecular weight of 300 000 g/mol; and hyaluronic acid in a mixture with carboxymethyl cellulose, polyethylene oxide and polyvinyl alcohol were prepared. Hyaluronic acid had the molecular weight of 150 000 g/mol, polyethylene oxide had the molecular weight of 300 000 g/mol, carboxymethylcellulose had the molecular weight of 250 000 g/mol, 16 % polyvinyl alcohol had the molecular weight of 63 000 g/mol. Table 2 below presents the substances and groups of substances in the way how they were added to the spinning solutions, including the concentrations.

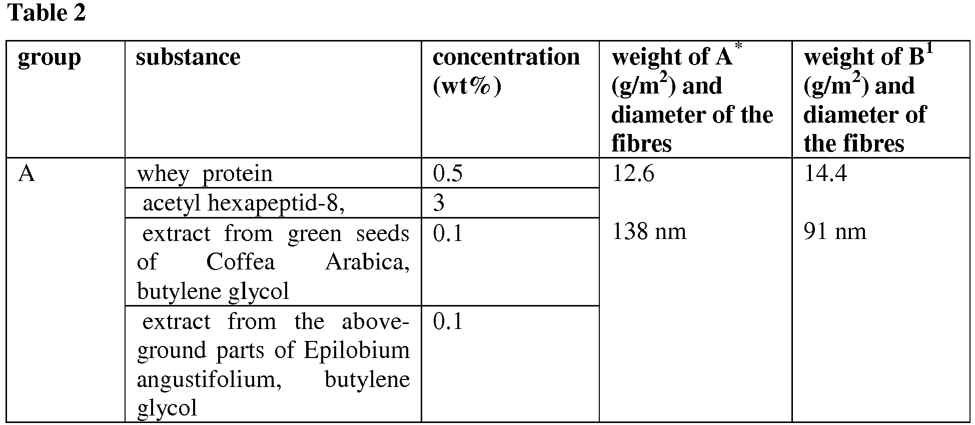

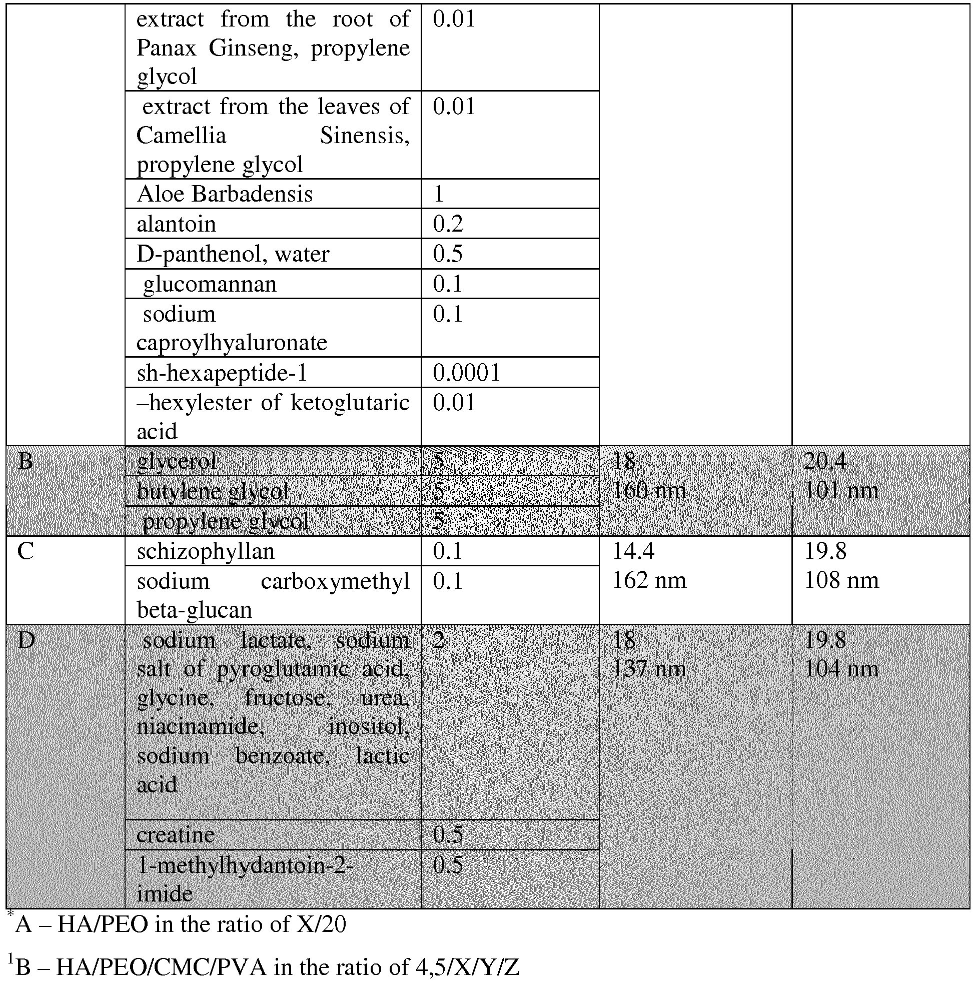

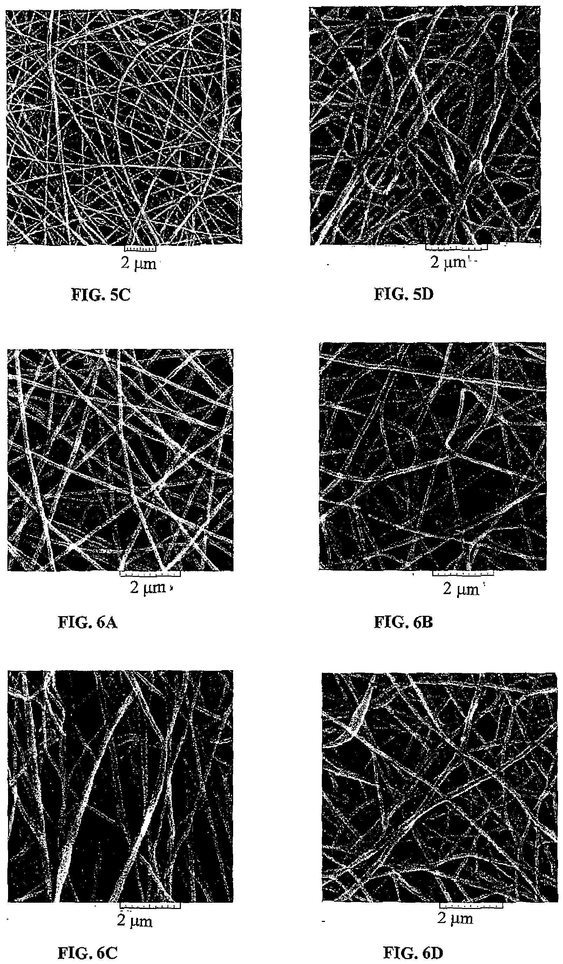

Eight samples were prepared.
Sample A contains in its composition HA/PEO and the substances from group A - see Table 2 above (see also Fig. 5A).
Sample B contains in its composition HA/PEO and the substances from groups A + B - see Table 2 above (see also Fig. 5B).
Sample C contains in its composition HA/PEO/CMC/PVA and the substances from group A - see Table 2 above (see also Fig. 5C).
Sample D contains in its composition HA/PEO/CMC/PVA and the substances from groups A + B - see Table 2 above (see also Fig. 5D).
Other two samples contained either HA/PEO and the substances from groups A + B + C or substances from groups A + B + C + D - see Table 2 above.
Other two samples contained either HA/PEO/CMC/PVA and either the substances from groups A + B + C or substances from groups A + B + C + D - see Table 2 above.

When spinning the cosmetically active substances, we started from the original 6 %wt. solution of hyaluronic acid and polyethylene oxide in the ratio of 80/20. The percentual fraction of PEO was retained on the level of 20 % of the dry matter and the HA fraction was reduced by the percentual presence of individual substances, i.e. the amount of hyaluronic acid was decreased. Upon incorporation of all of the components, the ratio of hyaluronic acid to polyethylene oxide was 60/20. From the initial mixture of the variant containing 4 %wt. of the dry matter where the percentual content of HA (4.5 %wt.) was maintained and from PVA, only percentual contents of solutions were subtracted - (extract from green seeds of Coffea Arabica, glycerol, and the like). From the initial concentration of CMC, percentual contents of liquids were subtracted and finally, from PEO, percentual contents of solids were subtracted. Therefore, in the end, the content of three carrier polymers was reduced. Production rates of these mixtures are presented in Table 4 as the production rate B. Fig. 5 shows nanofibrous layers of individual cosmetic mixtures.

### Example 16

Solutions of a solution of hyaluronic acid having the molecular weight of 80 000 g/mol and polyethylene oxide having the molecular weight of 400 000 g/mol; and hyaluronic acid in a mixture with carboxymethyl cellulose, polyethylene oxide and polyvinyl alcohol were prepared. Hyaluronic acid had the molecular weight of 80 000 g/mol, polyethylene oxideu had the molecular weight of 400 000 g/mol, carboxymethyl cellulose had the molecular weight of 150 000 g/mol, 16 % polyvinyl alcohol had the molecular weight of 91 000 g/mol. Table 3 below presents the substances and groups of substances in the way how they were added to the spinning solutions, including the concentrations.

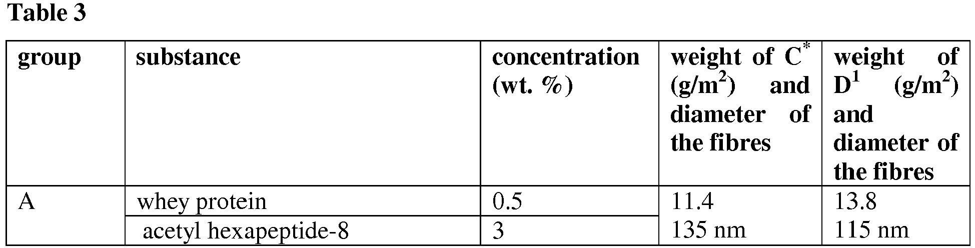

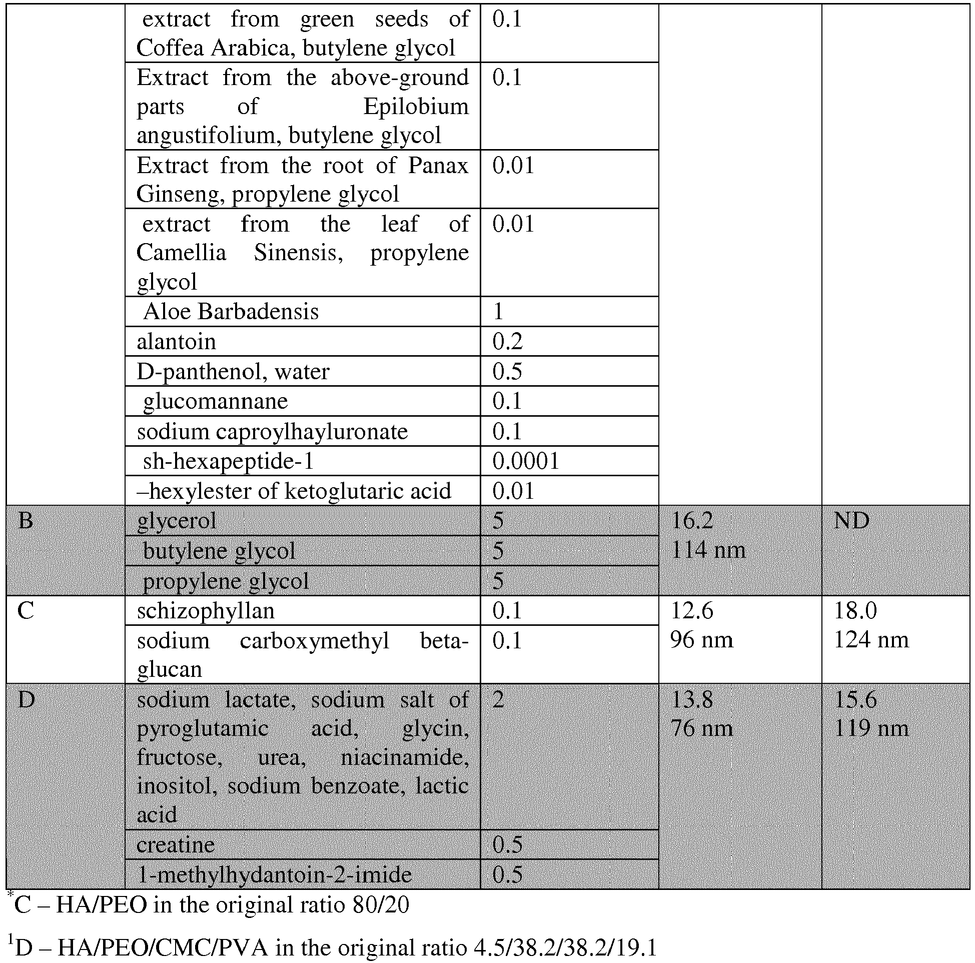

Eight samples were prepared.
Sample A contains in its composition HA/PEO and the substances from group A - see Table 3 above (see also Fig. 6A).
Sample B contains in its composition HA/PEO and the substances from groups A + B - see Table 3 above (see also Fig. 6B).
Sample C contains in its composition HA/PEO/CMC/PVA and the substances from group A - see Table 3 above (see also Fig. 6C).
Sample D contains in its composition HA/PEO/CMC/PVA and the substances from groups A + B - see Table 3 above (see also Fig. 6D).
Other two samples contained either HA/PEO and the substances from groups A + B + C or the substances from the groups A + B + C + D. - see Table 3 above.
Other two samples contained either HA/PEO/CMC/PVA and the substances from groups A + B + C or the substances from the groups A + B + C + D - see Table 3 above.

The ratio 80/20 was retained in the mixture HA/PEO, since the percentual contents of all substances were subtracted from the total dry matter and the remaining dry matter was then divided by the ratio 80/20. A similar approach was applied to the product containing two carrier polymers, hyaluronic acid and an additive which was carboxymethyl cellulose, where again the total sum of the percentual contents was subtracted from the total dry matter and the remainder was divided in the ratio HA/PEO/CMC/PVA 4.5/38.2/38.2/19.1. Production rates of these mixtures are presented in Table 3 as production rates C (HA/PEO) and D (HA/PEO/CMC/PVA).

### Example 17

A solution of 13.293 g of hyaluronic acid (HA) having the molecular weight of 83 200 g/mol and 1.8 g of polyethylene oxide having the molecular weight of 400 000 g/mol, 0,009 g whey protein, 0.9 g of propylene glycol, 1.8 g of an extract from the above-ground parts of Epilobium angustifolium, 0.18 g of Aloe Barbadensis, 0.018 g of sodium carboxymethyl beta-glucan and 280 g of water was prepared. Hyaluronic acid formed 73.1 % of the dry matter. The solution was homogenized for 14 hours at the temperature of 20 °C. The solution was spun to various types of support textiles. The production rates are presented in Table 4.

**Table 4 - List of support textiles, chemical compositions and production rates thereof**

| **type of the textile** | **composition** | **areal weight (g/m²)** | **diameter of the fibres (nm)** |
|---|---|---|---|
| non-woven textile made of nanofibres | polyamide + polyester | 7.2 | 72 |
| woven fabric in cloth binding | cotton | 7.8 | 88 |
| warp-knitted fabric | polyamide + elastan (70:30) | 9.0 | 105 |
| foil | low-density PE (fine) | 6.6 | 55 |
| warp-knitted fabric | polyester 100% | 9.6 | 85 |
| warp-knitted fabric | polyamide and elastan (81:19) | 3.0 | 79 |
| non-woven textile - 10 g/m² | polypropylene | 9.6 | 3.65 |
| foil | low-density polyethylene (PE) (rough) | 7.2 | 92 |
| warp-knitted fabric | viscose | 4.8 | 96 |
| jersey fabric | cellulose | 6.0 | 92 |
| woven textile | cellulose | 7.2 | 97 |
| warp-knitted fabric | polyamide monofil | 5.4 | 97 |
| non-woven textile - a hydrophobic modification | polyester | 13.2 | 115 |
| non-woven textile | polyester | 13.8 | 138 |
| non-woven textile | viscose + polyester | 7.2 | 121 |
| non-woven textile | polyurethane | 10.2 | 139 |
| non-woven textile - 25 g/m² | polypropylene | 13.2 | 154 |
| foil | polypropylene | 7.2 | 200 |
| foil | polyethylene | 9.6 | 179 |

### Example 18

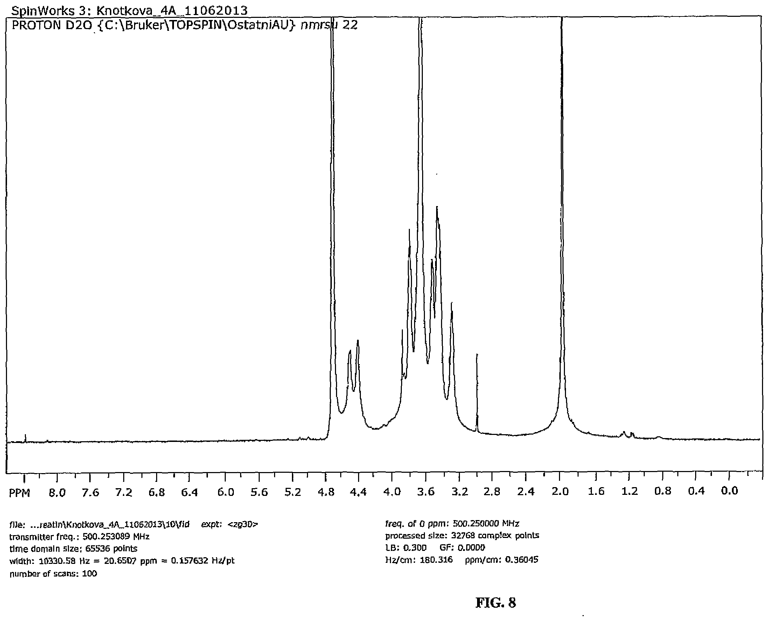

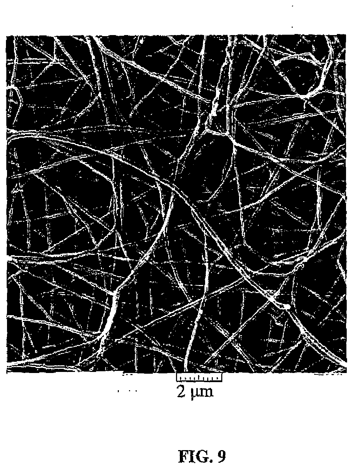

A solution of 2.385 g of hyaluronan (HA) having the molecular weight of 83 200 g/mol and 0.6 g of polyethylene oxide having the molecular weight of 400 000 g/mol, 0.015 g of creatine and 47 g of water was prepared. Hyaluronic acid formed 79.5 % of the dry matter. The solution was agitated in a shaking apparatus for 12 hours at the temperature of 25 °C. Thereafter it was spun. 10 mg of the sample was dissolved in deuterated water and H¹ NMR spectrum was measured (Fig. 8). The nanofibrous layer containing hyaluronic acid, polyethylene oxide and creatine is shown in Fig. 9.

### Example 19

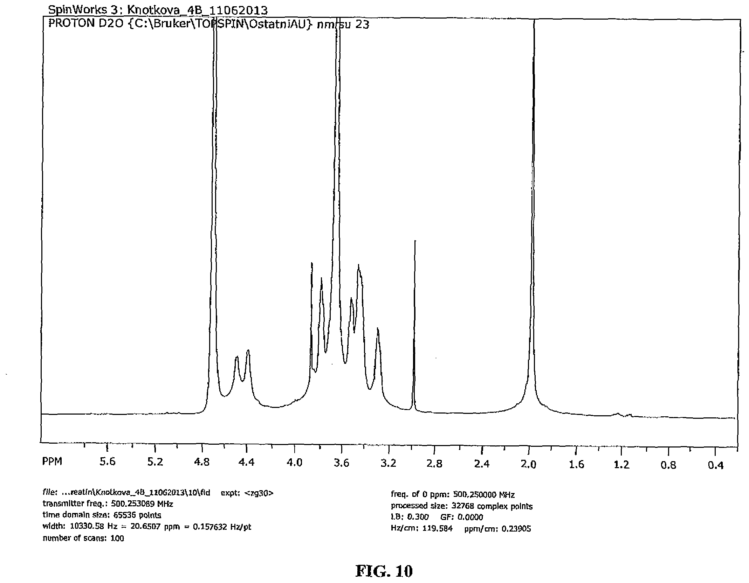

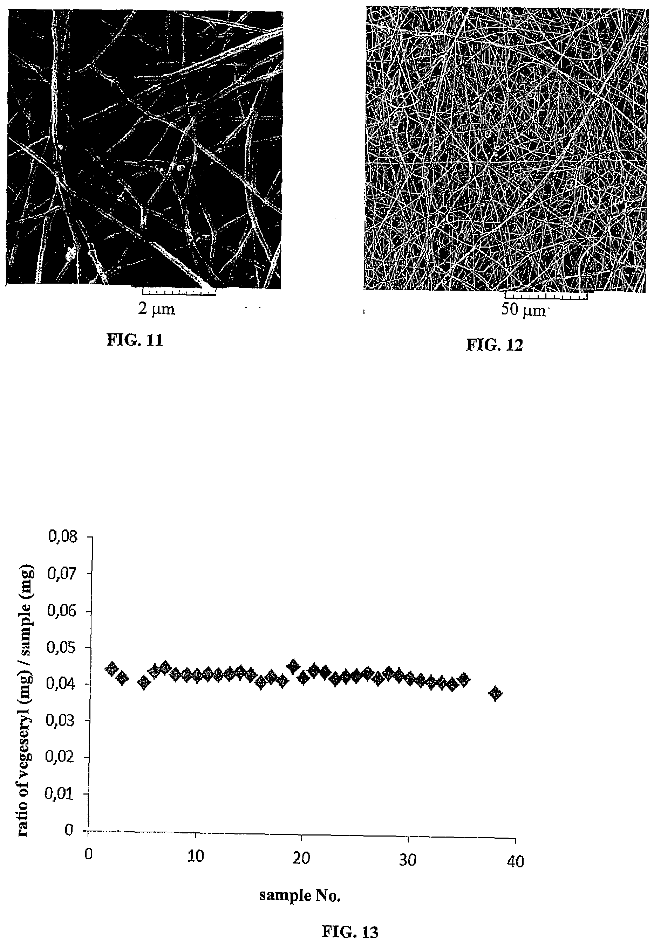

A solution of 2.34 g of hyaluronic acid (HA) having the molecular weight of 83 200 g/mol and 0.6 g of polyethylene oxide having the molecular weight of 400 000 g/mol, 0.06 g of creatine and 47 g of water was prepared. Hyaluronic acid formed 78 % of the dry matter. The solution was agitated in a shaking apparatus for 16 hours at the temperature of 20 °C. Thereafter it was spun. 10 mg of the sample was dissolved in deuterated water and H¹ NMR spectrum was measured (Fig. 10). The nanofibrous layer containing hyaluronic acid, polyethylene oxide and creatine is shown in Fig. 11.

### Example 20

A solution of 0.4 hyaluronic acid having the molecular weight of 10 000 g/mol containing polymeric micelles with pyridoxine palmitate, 0.1 g of polyethylene oxide having the molecular weight of 600 000 g/mol and 4.5 g of water was prepared. Hyaluronic acid formed 80 % of the dry matter. The solution was agitated in a shaking apparatus for 16 hours at the temperature of 25 °C. Thereafter it was spun. The areal weight of the nanofirbous layer is 10.71 g/m². The nanofibrous layer containing micelles with a lipidic component is shown in Fig. 12. The diameter of the fibres was 308 nm.

### Example 21

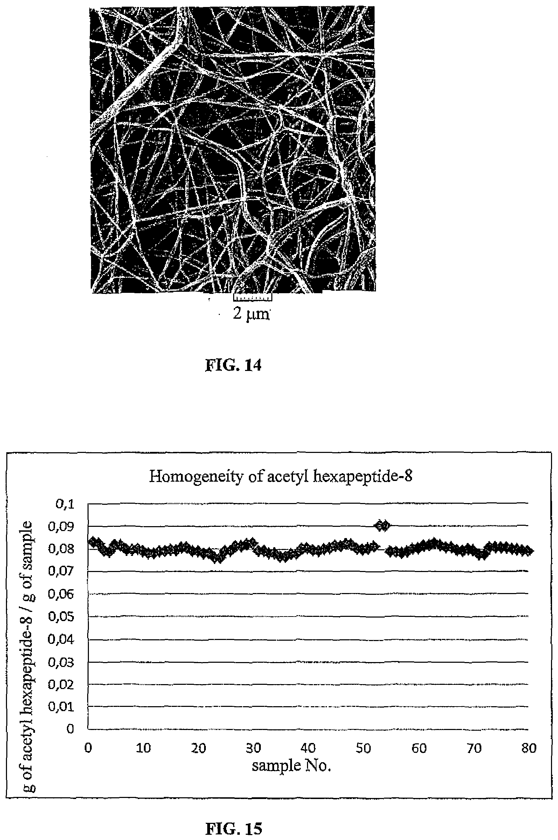

A solution of 4.5 g hyaluronic acid (HA) having the molecular weight of 83 200 g/mol and 1.2 g of polyethylene oxide having the molecular weight of 400 000 g/mol, 0.3 g of soya proteins and water and 94 g of water was prepared. Hyaluronic acid formed 69.23 % of the dry matter. The solution was agitated in a shaking apparatus for 12 hours at the temperature of 40 °C. Thereafter it was spun. The nanofibrous layer was cut to circles, in which the concentration of soya proteins and water was determined by means of UV-Vis. The initial concentration was 0.05 mg/mg of the sample. After spinning the concentration dropped to 0.045 mg/mg of the sample with a relative error of 3 %. This also proved the homogeneity of the spinning process (Fig. 13). Fig. 14 shows a nanofibrous layer obtained by spinning the mixture with soya proteins and water.

### Example 22

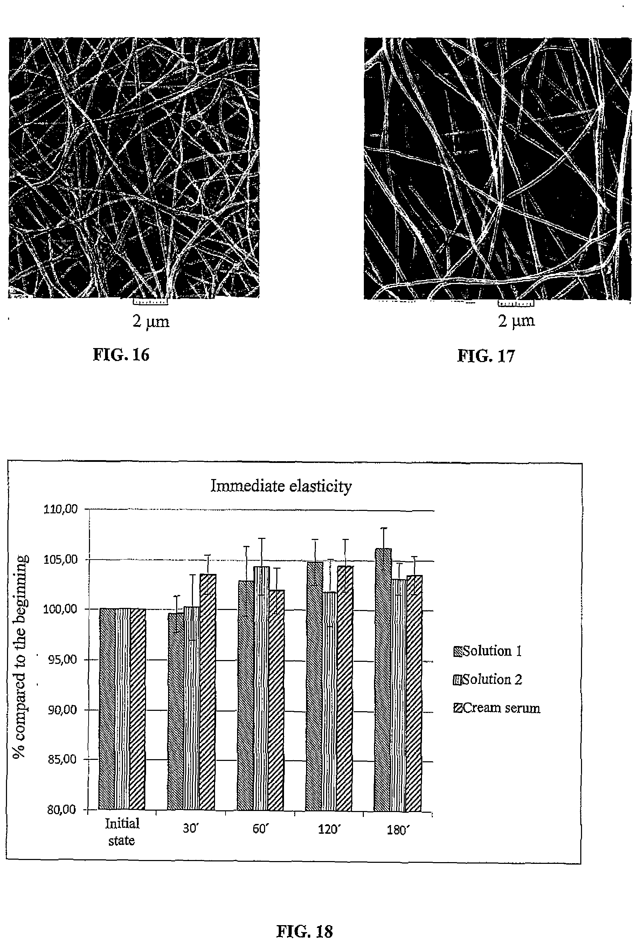

A solution of 8.4 g hyaluronic acid (HA) having the molecular weight of 83 200 g/mol and 2.4 g of polyethylene oxide having the molecular weight of 400 000 g/mol, 1.2 g of acetyl hexapeptide-8 and 188 g of water was prepared. Hyaluronic acid formed 70 % of the dry matter. The solution was agitated in a shaking apparatus for 14 hours at the temperature of 25 °C. Thereafter it was spun. The nanofibrous layer was cut to circles, in which the concentration of acetyl hexapeptide-8 was measured by means of LC-MS. The initial concentration was 0.1 mg/mg of the sample. After spinning the concentration dropped to 0.08 mg/mg of the sample with a relative error of 3 %. This also proved the homogeneity of the spinning process (Fig. 15). The nanofibrous layer is shown in Fig. 16.

### Example 23

A solution of 5.196 g of hyaluronic acid (HA) having the molecular weight of 83 200 g/mol and 6 g of polyethylene oxide having the molecular weight of 400 000 g/mol, 0.06 g whey protein, 0.6 g propylenglykolu, 1.2 g of an extract from the above-ground parts of Epilobium angustifolium, 0.12 g of Aloe Barbadensis, 0.012 g of carboxymethyl beta-glucan and 187 g of water was prepared. Hyaluronic acid formed 39.4 % of the dry matter. The solution was homogenized for 12 hours at the temperature of 20 °C. The solution was spun for various lengths of times (Table 5). The nanofibrous layer is shown in Fig. 17. The diameter of the fibres was 162 nm.

**Table 5 Various durations of the spinning process and the resulting weights**

| **time (min)** | **areal weight (g/m²)** |
|---|---|
| 1 | 0.2240 |
| 3 | 0.5920 |
| 5 | 0.8667 |
| 7 | 1.2827 |
| 10 | 1.7400 |
| 13 | 2.5640 |
| 15 | 3.0200 |
| 20 | 4.0880 |
| 250 | 46.8225 |

### Example 24

Comparative test of *in vivo* effects of the instant cosmetics in the form of a facial serum and cream serum on the skin elasticity during 3 and 24 hours after the application of the products.

A solution 1 of 4.02 g of hyaluronic acid (HA) having the molecular weight of 83 200 g/mol and 1.2 g of polyethylene oxide having the molecular weight of 400 000 g/mol, 0.06 g of plankton extract, 0.18 g of acetyl hexapeptide-8, 0.3 g of extract from the leaves of Camellia Sinensis, 0.06 g of Aloe Barbadensis, 0.18 g of soya protein was prepared. The solution was homogenized in a shaking apparatus for 12 hours. Thereafter, it was spun in a self-supporting way.

A solution 2 was prepared of 4.8 g of hyaluronic acid (HA) having the molecular weight of 83 200 g/mol and 1.2 g of polyethylene oxide having the molecular weight of 400 000 g/mol. The solution was homogenized in a shaking apparatus for 12 hours. Thereafter, it was spun in a self-supporting way.

A cream serum (emulsion of the o/w type) was prepared of 12 g of triglycerides of octanoic acid and decanoic acid, 5 g of the mixture of glyceryl monostearate and PEG 100 stearate, 2 g of the mixture of sodium acrylate, a copolymer of acryloyl dimethyl taurate, isohexadecane and polysorbate 80, 1.2 g of polyethylene oxide, 4.02 g of hyaluronic acid having the molecular weight of 83 200 g/mol, 0.06 g of plankton extract, 0.18 g of acetyl hexapeptide-8, 0.3 g of an extract from the leaves of Camellia Sinensis, 0.06 g of Aloe Barbadensis, 0.8 g of the mixture of benzylalcohol and dehydro acetic acid and 74.38 g of distilled water.

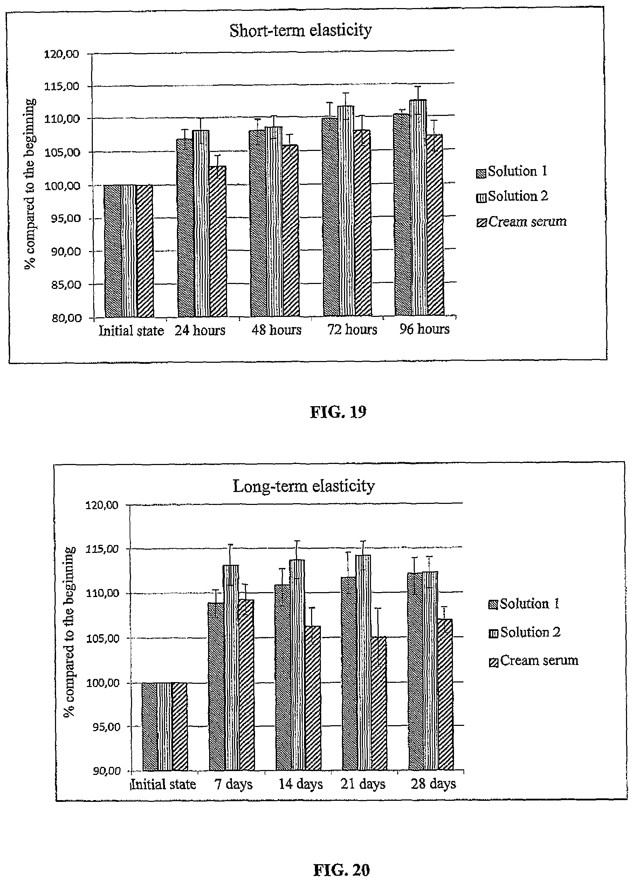

In order to monitor the effects of the dry facial serum and the cream serum on the skin elasticity, first the initial values of said parameter in the locations of the intended application of the products were measured. Then the spun samples of the solutions 1 and 2 and the cosmetic preparation having the weight of 5 to 10 mg were applied on a forearm. The spun samples were dissolved by means of a defined amount of water and then spred on the marked area, at the same time the cream serum was spread as well. The samples were not cleared off the skin. The effect of the products on the immediate elasticity of the skin was monitored in times 0.5, 1, 2 and 3 hours (Fig. 18) and on the short-term elasticity it was monitored for 4 days always in time 24 hours (Fig. 19) from the previous application of the products. The elasticity measurement was performed by means of the probe Cutometer® MPA 580.

### Example 25

Comparative test of *in vivo* effects of the instant cosmetics in the form of a facial serum and cream serum on the skin elasticity during 4 weeks of application of the products.

A solution 1 of 4.02 g of hyaluronic acid (HA) having the molecular weight of 83 200 g/mol and 1.2 g of polyethylene oxide having the molecular weight of 600 000 g/mol, 0.06 g of plankton extract, 0.18 g of acetyl hexapeptide-8, 0.3 g of extract from the leaves of Camellia Sinensis, 0.06 g of Aloe Barbadensis, 0.18 g of soya protein was prepared. The solution was homogenized in a shaking apparatus for 12 hours. Thereafter, it was spun in a self-supporting way.

A solution 2 was prepared of 4.8 g of hyaluronic acid (HA) having the molecular weight of 83 200 g/mol and 1.2 g of polyethylene oxide having the molecular weight of 600 000 g/mol. The solution was homogenized in a shaking apparatus for 12 hours. Thereafter, it was spun in a self-supporting way.

A cream serum (emulsion of the o/w type) was prepared of 12 g of triglycerides of octanoic acid and decanoic acid, 5 g of the mixture of glyceryl monostearate and PEG 100 stearate, 2 g of the mixture of sodium acrylate, a copolymer of acryloyl dimethyl taurate, isohexadecane and polysorbate 80, 1.2 g of polyethylene oxide, 4.02 g of hyaluronic acid having the molecular weight of 83 200 g/mol, 0.06 g of plankton extract, 0.18 g of acetyl hexapeptide-8, 0.3 g of an extract from the leaves of Camellia Sinensis, 0.06 g of Aloe Barbadensis, 0.8 g of the mixture of benzylalcohol and dehydro acetic acid and 74.38 g of distilled water.

In order to monitor the effects of the dry facial serum and the cream serum on the skin elasticity, first the initial values of said parameter in the locations of the intended application of the products were measured. Then the spun samples of the solutions 1 and 2 and the cosmetic preparation having the weight of 5 to 10 mg were applied on a forearm. The spun samples were dissolved by means of a defined amount of water and then spred on the marked area, at the same time the cream serum was spread as well. The samples were not cleared off the skin. The effect of the products on the long-term elasticity of the skin was monitored during a 4-week application in days 7, 14, 21 and 28 (Fig. 20). The elasticity measurement was performed by means of the probe Cutometer® MPA 580.

### Example 26

Comparative test of *in vivo* effects of the instant cosmetics in the form of a facial serum and cream serum on the amount of sebum on the skin within 2 hours from the application of the products.

A solution 1 of 4.02 g of hyaluronic acid (HA) having the molecular weight of 83 200 g/mol and 1.2 g of polyethylene oxide having the molecular weight of 400 000 g/mol, 0.06 g of plankton extract, 0.18 g of acetyl hexapeptide-8, 0.3 g of extract from the leaves of Camellia Sinensis, 0.06 g of Aloe Barbadensis, 0.18 g of soya protein was prepared. The solution was homogenized in a shaking apparatus for 12 hours. Thereafter, it was spun in a self-supporting way.

A solution 2 was prepared of 4.8 g of hyaluronic acid (HA) having the molecular weight of 83 200 g/mol and 1.2 g of polyethylene oxide having the molecular weight of 400 000 g/mol. The solution was homogenized in a shaking apparatus for 12 hours. Thereafter, it was spun in a self-supporting way.

A cosmetic preparation (emulsion of the o/w type) was prepared of 12 g of triglycerides of octanoic acid and decanoic acid, 5 g of the mixture of glyceryl monostearate and PEG 100 stearate, 2 g of the mixture of sodium acrylate, a copolymer of acryloyl dimethyl taurate, isohexadecane and polysorbate 80, 1.2 g of polyethylene oxide, 4.02 g of hyaluronic acid having the molecular weight of 83 200 g/mol, 0.06 g of plankton extract, 0.18 g of acetyl hexapeptide-8, 0.3 g of an extract from the leaves of Camellia Sinensis, 0.06 g of Aloe Barbadensis, 0.8 g of the mixture of benzylalcohol and dehydro acetic acid and 74.38 g of distilled water.

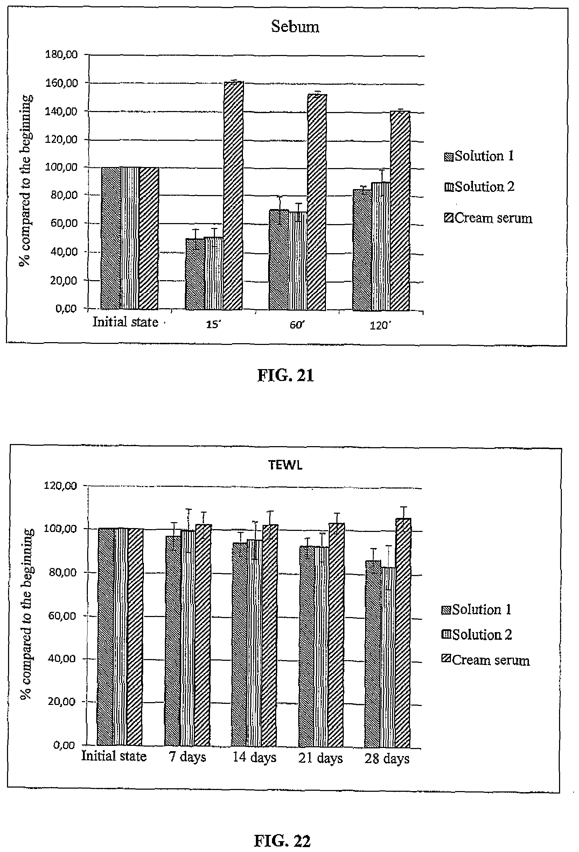

In order to monitor the effects of the dry facial serum and the cream serum on the sebum values on the skin, first the initial values of said parameter in the forehead area was measured. Then the spun samples of the solutions 1 and 2 and the cosmetic preparation having the weight of 5 to 10 mg were applied on the forehead. The spun samples were dissolved by means of a defined amount of water and then spred on the marked area, at the same time the cream serum was spread as well. The samples were not cleared off the skin. The values of sebum were monitored in times 15 minutes, 1 and 2 hours since the application of the products (Fig. 21). The sebum measurement was performed by means of the set Sebumeter®.

### Example 27

Comparative test of *in vivo* effects of the instant cosmetics in the form of a facial serum and cream serum on the loss of water from the skin (TEWL - Trans Epidermal Water Loss) during 4 weeks of application of the products.

A solution 1 of 4.02 g of hyaluronic acid (HA) having the molecular weight of 83 200 g/mol and 1.2 g of polyethylene oxide having the molecular weight of 400 000 g/mol, 0.06 g of plankton extract, 0.18 g of acetyl hexapeptide-8, 0.3 g of extract from the leaves of Camellia Sinensis, 0.06 g of Aloe Barbadensis, 0.18 g of soya protein was prepared. The solution was homogenized in a shaking apparatus for 12 hours. Thereafter, it was spun in a self-supporting way.

A solution 2 was prepared of 4.8 g of hyaluronic acid (HA) having the molecular weight of 83 200 g/mol and 1.2 g of polyethylene oxide having the molecular weight of 400 000 g/mol. The solution was homogenized in a shaking apparatus for 12 hours. Thereafter, it was spun in a self-supporting way.

A cosmetic preparation (emulsion of the o/w type) was prepared of 12 g of triglycerides of octanoic acid and decanoic acid, 5 g of the mixture of glyceryl monostearate and PEG 100 stearate, 2 g of the mixture of sodium acrylate, a copolymer of acryloyl dimethyl taurate, isohexadecane and polysorbate 80, 1.2 g of polyethylene oxide, 4.02 g of hyaluronic acid having the molecular weight of 83 200 g/mol, 0.06 g of plankton extract, 0.18 g of acetyl hexapeptide-8, 0.3 g of an extract from the leaves of Camellia Sinensis, 0.06 g of Aloe Barbadensis, 0.8 g of the mixture of benzylalcohol and dehydro acetic acid and 74.38 g of distilled water.

In order to monitor the effects of the dry facial serum and the cream serum on the water loss values of the skin (TEWL), first the initial values of said parameter in the locations of the intended application were measured. Then the spun samples of the solutions 1 and 2 and the cosmetic preparation having the weight of 5 to 10 mg were applied on the skin. The spun samples were dissolved by means of a defined amount of water and spred on the marked area, at the same time the cream serum was spread as well. The samples were not cleared off the skin. The TEWL values were monitored during a 4-week application of the products in days 7, 14, 21 and 28 (Fig. 22). The measurement was performed by means of the probe Tewameter® TM 3.

### Example 28

Comparative test of *in vivo* effects of the instant cosmetics in the form of a facial mask on the skin elasticity within 3 hours after the application of the products.

A solution 1 of 0.176 g of hyaluronic acid (HA) having the molecular weight of 83 200 g/mol, 1.528 g of polyethylene oxide having the molecular weight of 400 000 g/mol, 0.768 g of polyvinylalcohol and 1.528 g of carboxymethyl celullulose having the molecular weight of 250 000 g/mol was prepared. The solution was homogenized in a shaking apparatus for 12 hours. Thereafter, it was spun on a polypropylene non-woven textile.

A solution 2 was prepared of 0.176 g of hyaluronic acid (HA) having the molecular weight of 83 200 g/mol, 1.528 g of polyethylene oxide having the molecular weight of 400 000 g/mol, 0.768 g of polyvinylalcohol and 1.528 g of carboxymethyl cellulose having the molecular weight of 250 000 g/mol. The solution was homogenized in a shaking apparatus for 12 hours. Thereafter, it was spun in a self-supporting way.

A solution 3 was prepared of 4.8 g of hyaluronic acid (HA) having the molecular weight of 83 200 g/mol and 1.2 g of polyethylene oxide having the molecular weight of 400 000 g/mol. The solution was homogenized in a shaking apparatus for 12 hours. Thereafter, it was spun in a self-supporting way.

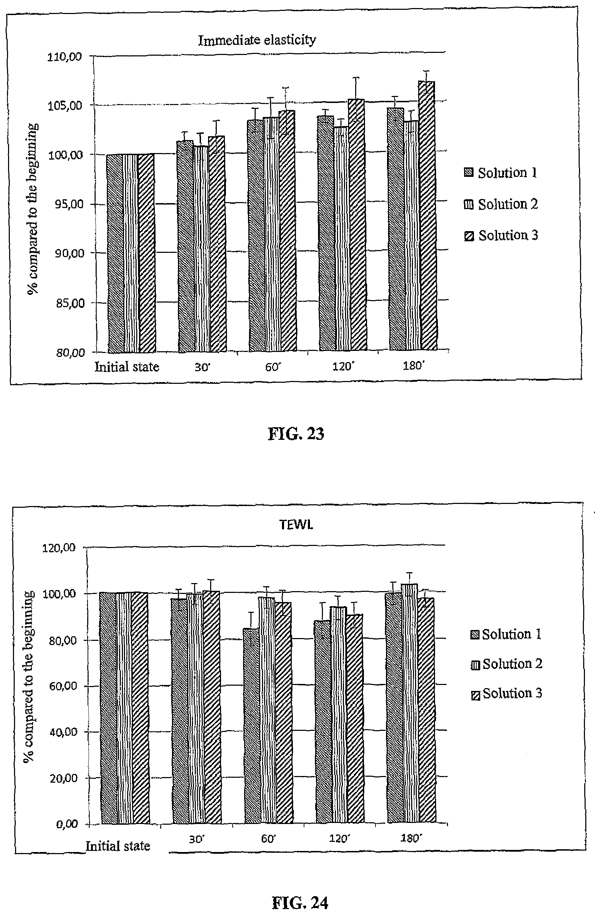

In order to monitor the effects of the dry facial mask on the skin elasticity, first the initial values of said parameter in the locations of the intended application of the products were measured. Then the spun samples of the solutions 1, 2 and 3 having the weight of 5 to 10 mg were applied on a forearm. The sample of the spun solution 1 was applied with the spun layer to the moistened skin for 15 minutes, then the product was cleared off by means of water. Samples of the spun solutions 2 and 3 were dissolved by means of a defined amount of water, spred on the marked area and were left on the skin for 10 minutes. The the products were removed by means of water. The effect of the products on the skin elasticity skin was monitored in times 0.5, 1, 2 and 3 hours since the application (Fig. 23). The elasticity measurement was performed by means of the probe Cutometer® MPA 580.

### Example 29

Comparative test of *in vivo* effects of the instant cosmetics in the form of a facial mask on the loss of water from the skin (TEWL - Trans Epidermal Water Loss) within 3 hours of application of the products.

A solution 1 of 0.176 g of hyaluronic acid (HA) having the molecular weight of 83 200 g/mol, 1.528 g of polyethylene oxide having the molecular weight of 400 000 g/mol, 0.768 g of polyvinylalcohol and 1.528 g of carboxymethyl celullulose having the molecular weight of 250 000 g/mol was prepared. The solution was homogenized in a shaking apparatus for 12 hours. Thereafter, it was spun on a polypropylene non-woven textile.

A solution 2 was prepared of 0.176 g of hyaluronic acid (HA) having the molecular weight of 83 200 g/mol, 1.528 g of polyethylene oxide having the molecular weight of 400 000 g/mol, 0.768 g of polyvinylalcohol and 1.528 g of carboxymethyl cellulose having the molecular weight of 250 000 g/mol. The solution was homogenized in a shaking apparatus for 12 hours. Thereafter, it was spun in a self-supporting way.

A solution 3 was prepared of 4.8 g of hyaluronic acid (HA) having the molecular weight of 83 200 g/mol and 1.2 g of polyethylene oxide having the molecular weight of 400 000 g/mol. The solution was homogenized in a shaking apparatus for 12 hours. Thereafter, it was spun in a self-supporting way.

In order to monitor the effects of the facial mask on the TEWL values, first the initial values of said parameter in the locations of the intended application were measured. Then the spun samples of the solutions 1, 2 and 3 having the weight of 5 to 10 mg were applied on a forearm in such a way that the sample of the spun solution 1 was laid with the spun layer to the moistened skin for 15 minutes, then the product together with the textile was cleared off by means of water. The samples of the spun solutions 2 and 3 were applied to the skin, dissolved by means of a defined amount of water, spred on the marked area and left on the skin for 10 minutes. Then the samples were cleared off the skin by means of water. The TEWL values were monitored in times 0.5, 1, 2 and 3 hours from the application (Fig. 24). The measurement was performed by means of the probe Tewameter® TM 300.

## Claims

1. A cosmetic composition in the form of nanofibres **characterized by that** it comprises nanofibres containing hyaluronic acid or a pharmaceutically acceptable salt thereof in the range of 50 to 90 wt.%, having the molecular weight within the range of 1x10⁴ to 3 x 10⁵ g/mol,
and at least one carrier polymer selected from the group consisting of polyethylene oxide, polyvinyl alcohol.

2. The composition according to claim 1, **characterized by that** the pharmaceutically acceptable salt is selected from the group including any of alkali metal ions, preferably Na⁺, K⁺.

3. The composition according to claim 1 or claim 2, **characterized by that** the carrier polymer is preferably polyethylene oxide.

4. The composition according to any of the preceding claims 1 to 3, **characterized by that** the diameter of the nanofibres is within the range 1 to 400 nm, preferably 20 to 300 nm, more preferably 50 to 200 nm.

5. The composition according to any of the preceding claims 1 to 4, **characterized by that** the nanofibres further contain a cosmetically active substance.

6. The composition according to claim 5, **characterized by that** the cosmetically active substance is selected from the group including
- water-soluble peptides, preferably selected from the group containing acetyl hexapeptide-8, sh-hexapeptide-1, whey protein or soya protein,
- water-soluble polysaccharides, preferably selected from the group containing schizophyllan, sodium carboxymethyl beta-glucane, sodium caproylhyaluronate or glucomanane,
- water-soluble plant extracts, preferably selected from the group containing an extract from the above-ground parts of Epilobium angustifolium, extract from green seeds of Coffea arabica, extract from the leaf of Camellia Sinensis, extract from the root of Panax Ginseng, extract from Aloe Barbadensis, plankton extract,
- water-soluble polyols, preferably selected from the group containing propylene glycol, butylene glycol, glycerol;
- water-soluble vitamins, preferably selected from the group containing D-panthenol, ethyl ester of ascorbic acid,
Micrococcus lyzate, creatine, 1-methylhydantoin-2-imide, sodium lactate, glycine, a sodium salt of pyroglutamic acid, fructose, urea, niacinamide, inositol, sodium benzoate or lactic acid.

7. The composition according to any of claims 1 to 6, **characterized by that** the nanofibres further contain an adjuvant, preferably carboxymethyl cellulose.

8. The composition according to any of claims 1 to 7, **characterized by that** the nanofibres further contain a lipophilic substance in the form of micelles.

9. The composition according to claim 8, **characterized by that** the lipophilic substance is preferably selected from the group containing a resin extract from the tree Commiphora Mukul, extract from Lavadula Stoechas in triglycerides of octanoic and decanoic acid, coenzyme Q10, ascorbic acid palmitate, pyridoxine dipalmitate, tocopheryl acetate, glycyrrhetinoic acid, cholesterol.

10. The composition according to any of claims 1 to 9 **characterized by that** it includes nanofibres containing hyaluronic acid or a pharmaceutically acceptable salt thereof and a mixture of polyethylene oxide, polyvinyl alcohol and carboxymethyl cellulose.

11. The composition according to claim 10 **characterized by that** the weight ratio of carboxymethyl cellulose, polyethylene oxide and polyvinyl alcohol in the mixture is from 1/1/0.01 to 1/1/1, preferably 1/1/0.5.

12. The composition according to any of the preceding claims 1 to 11 **characterized by that** the molecular weight of hyaluronic acid or the pharmaceutically acceptable salt thereof is within the range of 5x10⁴ to 1.5 x 10⁵ g/mol.

13. The composition according to any of the preceding claims 1 to 12 **characterized by that** the molecular weight of the carrier polymer is within the range from 1 x10⁴ to ̌9x10⁵ g/mol, wherein the molecular weight is preferably for polyethylene oxide within the range 3 x 10⁵ to 9 x 10⁵ g/mol or for polyvinyl alcohol within the range of 1 x 10⁴ to 4 x 10⁵ g/mol.

14. The composition according to claim 10 **characterized by that** the content of the polyethylene oxide is within the range 10 to 50 %wt. and for polyvinyl alcohol 5 to 25 %wt.

15. The composition according to any of the preceding claims 7 to 14 **characterized by that** the molecular weight of carboxymethyl cellulose is within the range 1 x 10⁵ to 4 x 10⁵ g/mol, preferably 1.5 x 10⁵ až 3 x 10⁵ g/mol.

16. The composition according to claim 15 **characterized by that** the content of carboxymethyl cellulose in the dry matter of nanofibres is within the range 0.001 to 50 %wt., preferably 10 to 40 %wt..

17. The composition according to any of the preceding claims 1 to 16 **characterized by that** it is in the form of a layer, wherein the areal weight of the layer is within the range of 1 to 50 g/m², preferably 4 to 20 g/m².

18. The composition according to any of the preceding claims 1 to 17 **characterized by that** it is in a water-soluble dry form.

19. The composition according to any of the preceding claims 17 or 18 **characterized by that** the layer lays on a support.

20. The composition according to claim 19 **characterized by that** the areal weight of the layer is 0.2 to 50 g/m², preferably 1.5 to 20 g/m².

21. The composition according to claim 19 or claim 20 **characterized by that** the support is a textile or a foil.

22. The composition according to claim 21 **characterized by that** the textile is selected from the group containing woven fabric, warp-knitted fabric, load knitted fabric, non-woven textile.

23. The composition according to claim 21 or claim 22 **characterized by that** the material of the textile is selected from the group comprising polyester, cellulose, polyurethane, polypropylene, polyethylene, viscose, polyamide or mixtures thereof.

24. The composition according to claim 21 **characterized by that** the foil material is selected from the group comprising aluminium, polyamide, polyester, polypropylene, polyethylene or mixtures thereof.

25. A method of production of the composition defined in any of claims 1 to 18, **characterized by that** a spinning solution is prepared, containing hyaluronic acid or a salt thereof and at least on carrier polymer in water, and then it is spun electrostatically.

26. The method according to claim 25, **characterized by that** a cosmetically active substance as defined in claim 6 is added to the spinning solution.

27. The method according to claim 25 or claim 26, **characterized by that** an adjuvant as defined in claim 7 is added to the spinning solution.

28. The method according to any of the preceding claims 25 to 27, **characterized by that** micelles containing a lipophilic substance or mixtures thereof, or a lipophilic substance alone or mixtures thereof, as defined in claim 8, are added to the spinning solution.

29. The method according to any of the preceding claims 25 to 28, **characterized by that** a spinning solution containing hyaluronic acid or a salt thereof and polyethylene oxide or containing hyaluronic acid and a mixture of polyethylene oxide, carboxymethyl cellulose and polyvinyl alcohol.

30. The method according to claim 29, **characterized by that** hyaluronic acid together with polyethylene oxide or together with the mixture of polymers polyethylene oxide, carboxymethyl cellulose and polyvinyl alcohol form 2 to 12 %wt., preferably 4 to 10 %wt. of the spinning solution.

31. The method of production of the composition according to claims 19 to 24, **characterized by that** the composition is applied to a support as defined in claims 21 to 24.

32. Use of the composition according to any of the preceding claims 1 to 24 in cosmetics.

33. Use of the composition according to any of claims 1 to 18 for the production of a facial mask, facial cream, facial serum.

34. Use of the composition according to any of claims 19 to 24 for the production of facial mask.

## Patentansprüche

1. Eine kosmetische Zusammensetzung in der Form von Nanofasern, **dadurch gekennzeichnet, dass** sie Nanofasern umfasst, die Hyaluronsäure oder ein pharmazeutisch akzeptierbares Salz derselben mit dem Molekulargewicht im Bereich von 1x10⁴ bis 3 x 10⁵ g/mol enthalten, wobei der Mengenanteil der Säure bzw. des Salzes im Bereich von 50 bis 90 Gew.-% liegt,
und die mindestens ein Trägerpolymer enthalten, das aus der Gruppe ausgewählt ist, die aus Polyethylenoxid und Polyvinylalkohol besteht.

2. Die Zusammensetzung nach Anspruch 1, **dadurch gekennzeichnet, dass** das pharmazeutisch akzeptierbare Salz aus der Gruppe ausgewählt ist, die die beliebige Ionen alkalischer Metalle, vorzugsweise Na⁺, K⁺, umfasst.

3. Die Zusammensetzung nach Anspruch 1 oder 2, **dadurch gekennzeichnet, dass** das Trägerpolymer vorzugsweise vom Polyethylenoxid gebildet ist.

4. Die Zusammensetzung nach einem der vorhergehenden Ansprüche 1 bis 3, **dadurch gekennzeichnet, dass** der Durchmesser der Nanofasern im Bereich von 1 bis 400 nm, vorzugsweise von 20 bis 300 nm, vorzüglicherweise von 50 bis 200 nm.

5. Die Zusammensetzung nach einem der vorhergehenden Ansprüche 1 bis 4, **dadurch gekennzeichnet, dass** die Nanofasern ferner eine kosmetisch aktive Substanz enthalten.

6. Die Zusammensetzung nach Anspruch 5, **dadurch gekennzeichnet, dass** die kosmetisch aktive Substanz aus der Gruppe ausgewählt ist, die folgende Substanzen umfasst:
- wasserlösliche Peptide, welche vorzugsweise aus der Gruppe ausgewählt sind, die Acetyl-Hexapeptid-8, sh-Hexapeptid-1, Weizenprotein oder Sojaprotein umfasst,
- wasserlösliche Polysaccharide, welche vorzugsweise aus der Gruppe ausgewählt sind, die Schizophyllan, Natriumcarboxymethyl-Beta-Glukan, Natrium-Kaproylhyaluronat oder Glukomannan umfasst,
- wasserlösliche Pflanzenextrakte, welche vorzugsweise aus der Gruppe ausgewählt sind, die einen Extrakt aus dem oberirdischen Teil der Pflanze Epilobium Angustifolium, einen Extrakt aus den grünen Samen der Pflanze Coffea Arabica, einen Extrakt aus der Blättern der Pflanze Camellia Sinensis, einen Extrakt aus der Wurzel der Pflanze Panax Ginseng, einen Extrakt aus der Pflanze Aloe Barbadensis sowie Planktonextrakt umfasst,
- wasserlösliche Polyole, welche vorzugsweise aus der Gruppe ausgewählt sind, die Polypropylen-Glykol, Butylen-Glykol und Glycerol umfasst;
- wassermischbare Vitamine, welche vorzugsweise aus der Gruppe ausgewählt sind, die D-Panthenol, Ethylestere der Ascorbinsäure, Micrococcus-Lysat, Kreatin, 1-Methylhydantoin-2-Imid, Natriumlaktat, Glycin, ein Natrium-Salz der Pyroglutamsäure, Fruktose, Harnstoff, Niacinamid, Inositol, Natriumbenzoat oder Milchsäure umfasst.

7. Die Zusammensetzung nach einem der Ansprüche 1 bis 6, **dadurch gekennzeichnet, dass** die Nanofasern ferner ein Adjuvans, vorzugsweise Carboxymethylcellulose, enthalten.

8. Die Zusammensetzung nach einem der Ansprüche 1 bis 7, **dadurch gekennzeichnet, dass** die Nanofasern ferner eine liophile Substanz, vorzugsweise in der Form von Mizellen, enthalten.

9. Die Zusammensetzung nach Anspruch 8, **dadurch gekennzeichnet, dass** die liophile Substanz vorzugsweise aus der Gruppe ausgewählt ist, die einen Extrakt aus dem Baum Commiphora Mukul, einen Extrakt aus der Pflanze Lavadula Stoechas in Triglyceriden der Octansäure und der Decansäure, Coenzym Q10, Ascorbinsäurepalmitat, Pyridoxindipalmitat, Tocopherolacetat, Glycyrrhetinsäure und Cholesterol umfasst.

10. Die Zusammensetzung nach einem der Ansprüche 1 bis 9, **dadurch gekennzeichnet, dass** sie Nanofasern umfasst, die Hyaluronsäure oder ein pharmazeutisch akzeptierbares Salz derselben sowie ein Gemisch von Polyethylenoxid, Polyvinylalkohol und Carboxymethylcellulose enthalten.

11. Die Zusammensetzung nach Anspruch 10, **dadurch gekennzeichnet, dass** das Gewichtsverhältnis von Carboxymethylcellulosem, Polyethylenoxid und Polyvinylalkohol in dem Gemisch im Bereich von 1/1/0,01 bis 1/1/1 liegt und vorzugsweise 1/1/0,5 beträgt.

12. Die Zusammensetzung nach einem der vorhergehenden Ansprüche 1 bis 11, **dadurch gekennzeichnet, dass** das Molekulargewicht der Hyaluronsäure oder des pharmazeutisch akzeptierbaren Salzes derselben im Bereich von 5x10⁴ bis 1,5 x 10⁵ g/mol liegt.

13. Die Zusammensetzung nach einem der vorhergehenden Ansprüche 1 bis 12 **dadurch gekennzeichnet, dass** das Molekulargewicht des Trägerpolymers im Bereich von 1 x10⁴ bis 9x10⁵ g/mol liegt, wobei das Molekulargewicht für Polyethylenoxid vorzugsweise im Bereich von 3 x 10⁵ bis 9 x 10⁵ g/mol oder für Polyvinylalkohol vorzugsweise im Bereich von 1 x10⁴ bis 4 x 10⁵ g/mol liegt.

14. Die Zusammensetzung nach Anspruch 10 **dadurch gekennzeichnet, dass** der Gehalt an Polyethylenoxid im Bereich von 10 bis 50 Gew-% und der Gehalt an Polyvinylalkohol im Bereich von 5 bis 25 Gew.-% liegt.

15. Die Zusammensetzung nach einem der vorhergehenden Ansprüche 7 bis 14 **dadurch gekennzeichnet, dass** das Molekulargewicht der Carboxymethylcellulose im Bereich von 1 x 10⁵ bis 4 x 10⁵ g/mol, vorzugsweise von 1,5 x 10⁵ bis 3 x 10⁵ g/mol, liegt.

16. Die Zusammensetzung nach dem Anspruch 15 **dadurch gekennzeichnet, dass** der Gehalt an Carboxymethylcellulose in der Trockenmasse der Nanofasern im Bereich von 0,001 bis 50 % Gew.-%, vorzugsweise von 10 bis 40 Gew.-%, liegt.

17. Die Zusammensetzung nach einem der vorhergehenden Ansprüche 1 bis 16 **dadurch gekennzeichnet, dass** sie in der Form einer Schicht vorliegt, wobei das Flächengewicht der Schicht im Bereich von 1 bis 50 g/m², vorzugsweise von 4 bis 20 g/m², liegt.

18. Die Zusammensetzung nach einem der vorhergehenden Ansprüche 1 bis 17, **dadurch gekennzeichnet, dass** sie in der wasserlöslichen trockenen Form vorliegt.

19. Die Zusammensetzung nach einem der vorhergehenden Ansprüche 17 oder 18, **dadurch gekennzeichnet, dass** die Schicht auf einem tragenden Untergrund beruht.

20. Die Zusammensetzung nach Anspruch 19 **dadurch gekennzeichnet, dass** das Flächengewicht der Schicht 0,2 bis 50 g/m², vorzugsweise 1,5 bis 20 g/m², beträgt.

21. Die Zusammensetzung nach Anspruch 19 oder 20, **dadurch gekennzeichnet, dass** der tragende Untergrund von einer Textilie oder Folie gebildet ist.

22. Die Zusammensetzung nach Anspruch 21 **dadurch gekennzeichnet, dass** die Textilie aus der Gruppe ausgewählt ist, die eine gewebte Textilie, ein Kettgewirk, ein Gestrick und eine ungewebte Textilie umfasst.

23. Die Zusammensetzung nach Anspruch 21 oder Anspruch 22, **dadurch gekennzeichnet, dass** das Material der Textilie aus der Gruppe ausgewählt ist, die Polyester, Cellulose, Polyurethan, Polypropylen, Polyethylen, Viskose, Polyamid oder deren Gemische umfasst.

24. Die Zusammensetzung nach Anspruch 21, **dadurch gekennzeichnet, dass** das Material der Folie aus der Gruppe ausgewählt ist, die Aluminium, Polyamid, Polyester, Polypropylen, Polyethylen oder deren Gemische umfasst.

25. Verfahren zur Herstellung der in einem der Ansprüche 1 bis 18 definierten Zusammensetzung, **dadurch gekennzeichnet, dass** eine zu verspinnende Lösung, die Hyaluronsäure oder deren Salzform sowie mindestens ein Trägerpolymer im Wasser enthält, vorbereitet und anschliessend elektrostatisch versponnen wird.

26. Verfahren nach Anspruch 25, **dadurch gekennzeichnet, dass** das der zu verspinnenden Lösung eine im Anspruch 6 definierte kosmetisch aktive Substanz zugegeben wird.

27. Verfahren nach Anspruch 25 oder Anspruch 26, **dadurch gekennzeichnet, dass** das der zu verspinnenden Lösung ein im Anspruch 7 definiertes Adjuvans zugegeben wird.

28. Verfahren nach einem der vorhergehenden Ansprüche 25 bis 27, **dadurch gekennzeichnet, dass** der zu verspinnenden Lösung Mizellen zugegeben werden, die eine im Anspruch 8 definierte liophile Substanz oder ein Gemisch von derartigen liophilen Substanzen enthalten, bzw. unmittelbar eine derartige liophile Substanz oder ein Gemisch von derartigen liophilen Substanzen zugegeben wird.

29. Verfahren nach einem der vorhergehenden Ansprüche 25 bis 28, **dadurch gekennzeichnet, dass** eine zu verspinnende Lösung vorbereitet wird, die Hyaluronsäure oder deren Salzform mit Polyethylenoxid oder Hyaluronsäure mit einem Gemisch von Polyethylenoxid, Carboxymethylcellulose und Polyvinylalkohol enthält.

30. Verfahren nach Anspruch 29, **dadurch gekennzeichnet, dass** die Hyaluronsäure zusammen mit Polyethylenoxid oder zusammen mit dem Gemisch von Polymeren, die Polyethylenoxid, Carboxymethylcellulose und Polyvinylalkohol 2 bis 12 Gew.-%, vorzugsweise 4 bis 10 Gew.-% der zu verspinnenden Lösung bildet.

31. Verfahren zur Herstellung der Zusammensetzung nach einem der Ansprüche 19 bis 24, **dadurch gekennzeichnet, dass** die Zusammensetzung auf einen in Ansprüchen 21 bis 24 definierten tragenden Untergrund aufgebracht wird.

32. Anwendung der Zusammensetzung nach nach einem der vorhergehenden Ansprüche 1 bis 24 in der Kosmetik.

33. Anwendung der Zusammensetzung nach nach einem der Ansprüche 1 bis 18 zur Herstellung von Gesichtsmasken, Gesichtscremen und Gesichtsseren.

34. Anwendung der Zusammensetzung nach nach einem der Ansprüche 19 bis 24 zur Herstellung von Gesichtsmasken.

## Revendications

1. Composition cosmétique sous forme de nanofibres **caractérisée en ce qu'**elle comprend des nanofibres contenant de l'acide hyaluronique ou un de ses sels pharmaceutiquement acceptables dans la gamme de 50 à 90% en poids, ayant un poids moléculaire compris entre 1x10⁴ et 3 x 10⁵ g/mol,
et au moins un polymère support choisi dans le groupe constitué par l'oxyde de polyéthylène, l'alcool polyvinylique.

2. La composition selon la revendication 1, **caractérisée en ce que** le sel pharmaceutiquement acceptable est choisi dans le groupe comprenant l'un quelconque des ions de métal alcalin, de préférence Na⁺, K⁺.

3. La composition selon la revendication 1 ou la revendication 2, **caractérisée en ce que** le polymère support est de préférence l'oxyde de polyéthylène.

4. La composition selon l'une quelconque des revendications précédentes 1 à 3, **caractérisée en ce que** le diamètre des nanofibres est compris entre 1 et 400 nm, de préférence entre 20 et 300 nm, plus préférentiellement entre 50 et 200 nm.

5. La composition selon l'une quelconque des revendications précédentes 1 à 4, **caractérisée en ce que** les nanofibres contiennent en outre une substance cosmétiquement active.

6. La composition selon la revendication 5, **caractérisée en ce que** la substance cosmétiquement active est choisie dans le groupe comprenant
- des peptides hydrosolubles, de préférence choisis dans le groupe comprenant l'acétyl hexapeptide-8, le sh-hexapeptide-1, la protéine de lactosérum ou la protéine de soja,
- des polysaccharides hydrosolubles, de préférence choisis dans le groupe comprenant le schizophyllan, le carboxyméthylbêta-glucane sodique, le caproylhyaluronate de sodium ou le glucomanane,
- des extraits végétaux hydrosolubles, de préférence choisis dans le groupe comprenant un extrait des parties aériennes d'Epilobium angustifolium, un extrait de graines vertes de Coffea arabica, un extrait de feuilles de Camellia Sinensis, uns extrait de racines de Panax Ginseng, un extraits d'Aloe Barbadensis, un extrait de plancton,
- des polyols hydrosolubles, de préférence choisis dans le groupe comprenant le propylène glycol, le butylène glycol, le glycérol;
- des vitamines hydrosolubles, de préférence choisies dans le groupe comprenant le D-panthénol, ester éthylique de l'acide ascorbique,
Lactate de micrococcus, créatine, 1-méthylhydantoïne-2-imide, lactate de sodium, glycine, un sel de sodium de l'acide pyroglutamique, fructose, urée, niacinamide, inositol, benzoate de sodium ou acide lactique.

7. La composition selon l'une quelconque des revendications 1 à 6, **caractérisée en ce que** les nanofibres contiennent en outre un adjuvant, de préférence la carboxyméthylcellulose.

8. La composition selon l'une quelconque des revendications 1 à 7, **caractérisée en ce que** les nanofibres contiennent en outre une substance lipophile sous forme de micelles.

9. La composition selon la revendication 8, **caractérisée en ce que** la substance lipophile est de préférence choisie dans le groupe contenant un extrait de résine de l'arbre Commiphora Mukul, un extrait de Lavadula Stoechas dans des triglycérides de l'acide octanoïque et décanoïque, coenzyme Q10, palmitate de l'acide ascorbique, dipalmitate de pyridoxine, acétate de tocophéryle, l'acide glycyrrhétinoïque, cholestérol.

10. La composition selon l'une quelconque des revendications 1 à 9 **caractérisée en ce qu'**elle comprend des nanofibres contenant de l'acide hyaluronique ou un sel pharmaceutiquement acceptable de celui-ci et un mélange d'oxyde de polyéthylène, d'alcool polyvinylique et de carboxyméthylcellulose.

11. La composition selon la revendication 10 **caractérisée en ce que** le rapport en poids de la carboxyméthylcellulose, de l'oxyde de polyéthylène et de l'alcool polyvinylique dans le mélange est compris entre 1/1 / 0,01 et 1/1/1, de préférence 1/1 / 0,5.

12. La composition selon l'une quelconque des revendications précédentes 1 à 11, **caractérisée en ce que** le poids moléculaire de l'acide hyaluronique ou de son sel pharmaceutiquement acceptable est compris dans l'intervalle de 5 x 10⁴ à 1,5 x 10⁵ g / mol.

13. La composition selon l'une quelconque des revendications précédentes 1 à 12, **caractérisée en ce que** la masse moléculaire du polymère support est comprise entre 1 x 10⁴ et 9 x 10⁵ g / mol, la masse moléculaire étant de préférence pour l'oxyde de polyéthylène dans l'intervalle 3 x 10⁵ à 9 x 10⁵ g / mol ou pour l'alcool polyvinylique dans l'intervalle de 1 x 10⁴ à 4 x 10⁵ g / mol.

14. La composition selon la revendication 10 **caractérisée en ce que** la teneur en oxyde de polyéthylène est comprise entre 10 et 50% en poids. et pour l'alcool polyvinylique, de 5 à 25% en poids.

15. La composition selon l'une quelconque des revendications précédentes 7 à 14 **caractérisée en ce que** le poids moléculaire de la carboxyméthylcellulose est compris entre 1 x 10⁵ et 4 x 10⁵ g / mol, de préférence 1,5 x 10⁵ až 3 x 10⁵ g / mol.

16. La composition selon la revendication 15 **caractérisée en ce que** la teneur en carboxyméthylcellulose dans la matière sèche des nanofibres est comprise entre 0,001 et 50% en poids, de préférence entre 10 et 40% en poids.

17. La composition selon l'une quelconque des revendications précédentes 1 à 16, **caractérisée en ce qu'**elle se présente sous la forme d'une couche, dans laquelle le poids surfacique de la couche est compris entre 1 et 50 g/m², de préférence entre 4 et 20 g/m².

18. La composition selon l'une quelconque des revendications précédentes 1 à 17 **caractérisée en ce qu'**elle est sous une forme sèche soluble dans l'eau.

19. La composition selon l'une quelconque des revendications précédentes 17 ou 18, **caractérisée en ce que** la couche repose sur un support.

20. La composition selon la revendication 19, **caractérisée en ce que** le poids surfacique de la couche est de 0,2 à 50 g/m², de préférence de 1,5 à 20 g/m².

21. La composition selon la revendication 19 ou la revendication 20, **caractérisée en ce que** le support est un textile ou une feuille.

22. La composition selon la revendication 21, **caractérisée en ce que** le textile est choisi dans le groupe comprenant tissu tissée, tissu tricoté à chaîne, tissu tricoté chargé, textile non tissé.

23. La composition selon la revendication 21 ou la revendication 22, **caractérisée en ce que** le matériau du textile est choisi dans le groupe comprenant le polyester, la cellulose, le polyuréthane, le polypropylène, le polyéthylène, la viscose, le polyamide ou leurs mélanges.

24. La composition selon la revendication 21, **caractérisée en ce que** le matériau de la feuille est choisi dans le groupe comprenant l'aluminium, le polyamide, le polyester, le polypropylène, le polyéthylène ou leurs mélanges.

25. Procédé de production de la composition définie dans l'une quelconque des revendications 1 à 18, **caractérisé en ce qu'**on prépare une solution de filage contenant de l'acide hyaluronique ou un sel de celui-ci et au moins un polymère support dans l'eau, puis on le file électrostatiquement.

26. Le procédé selon la revendication 25, **caractérisé en ce qu'**une substance cosmétiquement active telle que définie dans la revendication 6 est ajoutée à la solution de filage.

27. Le procédé selon la revendication 25 ou la revendication 26, **caractérisé en ce qu'**un adjuvant tel que défini dans la revendication 7 est ajouté à la solution de filage.

28. Le procédé selon l'une quelconque des revendications précédentes 25 à 27, **caractérisé en ce que** des micelles contenant une substance lipophile ou des mélanges de celles-ci, ou une substance lipophile seule ou leurs mélanges, tels que définis dans la revendication 8, sont ajoutées à la solution de filage.

29. Le procédé selon l'une quelconque des revendications 25 à 28, **caractérisé en ce qu'**on utilise une solution de filage contenant de l'acide hyaluronique ou un sel de ce-ci et de l'oxyde de polyéthylène ou contenant de l'acide hyaluronique et un mélange de l'oxyde de polyéthylène, carboxyméthylcellulose et alcool polyvinylique.

30. Le procédé selon la revendication 29, **caractérisé en ce que** l'acide hyaluronique avec l'oxyde de polyéthylène ou avec le mélange de polymères l'oxyde de polyéthylène, carboxyméthylcellulose et alcool polyvinylique forment de 2 à 12% en poids, de préférence de 4 à 10% en poids. de la solution de filage.

31. Le procédé de production de la composition selon les revendications 19 à 24, **caractérisé en ce que** la composition est appliquée sur un support tel que défini dans les revendications 21 à 24.

32. Utilisation de la composition selon l'une quelconque des revendications précédentes 1 à 24 en cosmétique.

33. Utilisation de la composition selon l'une quelconque des revendications 1 à 18 pour la production d'un masque facial, d'une crème pour le visage, d'un sérum facial.

34. Utilisation de la composition selon l'une quelconque des revendications 19 à 24 pour la production d'un masque facial.
